(19) Europäisches Patentamt
European Patent Office
Office européen des brevets

(11) EP 1 721 600 A1

(12) EUROPÄISCHE PATENTANMELDUNG

(43) Veröffentlichungstag:
**15.11.2006 Patentblatt 2006/46**

(51) Int Cl.:
***A61K 8/35*** *(2006.01)* ***A61Q 17/04*** *(2006.01)*

(21) Anmeldenummer: **06112463.2**

(22) Anmeldetag: **11.04.2006**

(84) Benannte Vertragsstaaten:
**AT BE BG CH CY CZ DE DK EE ES FI FR GB GR HU IE IS IT LI LT LU LV MC NL PL PT RO SE SI SK TR**
Benannte Erstreckungsstaaten:
**AL BA HR MK YU**

(30) Priorität: **09.05.2005 DE 102005021913**

(71) Anmelder: **Beiersdorf AG**
**20253 Hamburg (DE)**

(72) Erfinder:
- **Bürger, Annette**
  **22301 Hamburg (DE)**
- **Batzer, Jan**
  **22459 Hamburg (DE)**
- **Blatt, Thomas**
  **22880 Wedel (DE)**
- **Wendel, Volker**
  **60529 Frankfurt/Main (DE)**
- **Wolber, Rainer**
  **22397 Hamburg (DE)**

(54) **Sonnenschutzkit und Verfahren zur Pigmentierungsangleichung**

(57) Verwendung von kosmetischen und dermatologischen Zubereitungen, die mindestens zwei unterschiedliche UV-Absorptionsverhalten aufweisen zur Färbung der Haut von mehrzelligen Organismen, insbesondere der Haut von Mensch und Tier, insbesondere zur Farbangleichung von unterschiedlich pigmentierten Hautstellen.



Printed by Jouve, 75001 PARIS (FR)

## Beschreibung

Die vorliegende Erfindung betrifft die Verwendung von mindestens zwei Lichtschutzzubereitungen zur Angleichung der Färbung normaler Haut an nicht normal pigmentierte Haut, insbesondere Altersflecken sowie großflächige Hyperpigmentierungen wie z. B. Melasma und postinflammatorische Hyperpigmentierungen.

Der Trend weg von der vornehmen Blässe hin zur "gesunden, sportlich braunen Haut" ist seit Jahren ungebrochen. Um diese zu erzielen, setzen die Menschen ihre Haut der Sonnenstrahlung aus, da diese eine Pigmentbildung im Sinne einer Melaninbildung hervorruft. Die ultraviolette Strahlung des Sonnenlichtes hat jedoch eine schädigende Wirkung auf die Haut. Neben der akuten Schädigung (Sonnenbrand) treten Langzeitschäden wie ein erhöhtes Risiko an Hautkrebs zu erkranken bei übermäßiger Bestrahlung mit Licht aus dem UVB-Bereich (Wellenlänge: 280-320 nm) auf. Die übermäßige Einwirkung der UVB- und UVA-Strahlung (Wellenlänge: 320-400 nm) führt darüber hinaus zu einer Schwächung der elastischen und kollagenen Fasern des Bindegewebes. Dies führt zu zahlreichen phototoxischen und photoallergischen Reaktionen und hat eine vorzeitige Hautalterung zur Folge.

Die natürliche Bräunung der Haut durch Sonnenstrahlung wird verursacht durch die Bildung von braunen Farbpigmenten in der Haut. Die Epidermis enthält in ihrer untersten Schicht, der Basalschicht, neben den Basalzellen einzelne pigmentbildende Zellen, die Melanocyten. Durch UV-Licht wird in diesen Zellen die Produktion von Melanin angeregt, das in die Keratinozyten und letztlich in die Korneozyten (Hornzellen) transportiert und dort als braune Hautfarbe sichtbar wird. Diese von der Aminosäure Tyrosin ausgehende Pigmentbildung wird überwiegend durch UVB-Strahlung initiiert und als "indirekte Pigmentierung" bezeichnet. Ihre Entwicklung läuft über mehrere Tage; die so erhaltene Sonnenbräune besteht über einige Wochen.

Jeder Mensch zeigt ein persönliches Hautbild. Insbesondere die Oberflächenstruktur der Haut ist einmalig, dadurch ist es möglich Personen anhand ihrer Fingerabdrücke zu identifizieren.

Auch die Färbung der Haut ist sehr unterschiedlich. Es gibt natürliche Hautpigmentierungsstörungen, wie zum Beispiel Sommersprossen (Lentigo aestiva), Altersflecke (Lentigo senilis, Keratosis senilis), Leberflecke (Lentigo simplex, Lentigo juvenilis, Naevi) und unnatürliche bzw. krankhafte Hautpigmentierungsstörungen wie zum Beispiel Lentigo maligna (Dubreuilh-Hutchinson Krankheit), Vitiligo, Narben von beispielsweise Operationen, Verletzungen oder Verbrennungen (postinflammatorische Hyperpigmentierungen), Melasma, unregelmäßige Pigmentierung der Haut mit lokal hyper- und/oder hypopigmentierten Bereichen *(uneven skin tone)* infolge äußerer Einflüsse (insbesondere nach chronischer UV-Belastung).

Im Rahmen der zuvor beschriebenen Alterungsprozesse kommt es auch zur Entstehung von Altersflecken in UV-Exponierten Arealen. Insbesondere die dunkleren Phototypen (Asiaten, Hispanics, auch Afrikaner) leiden häufig unter großflächigen Hyperpigmentierungen (beispielsweise Melasma) oder unter postinflammatorischen Hyperpigmentierungen. Die letztgenannten treten besonders beim männlichen Teil der Bevölkerung auf, wenn diese unter Verwachsungen der Barthaare leiden, sowie im Rahmen einer schweren Akne. Viele dieser Hyperpigmentierungen werden unter Einfluss von UV-Licht noch verstärkt.

Unterschiedliche Hautpigmentierung, insbesondere 'fleckige' Haut ("*uneven skin tone")* und auch sommersprossige Haut stellt für viele Menschen einen belastenden Mangel dar. Durch die im Alter zunehmende Zahl von Altersflecken nimmt das subjektive Gefühl des 'Altwerdens' bzw. 'Altseins' zu. Dieser psychologische Druck kann zu einer Beeinträchtigung des Lebenswandels führen.

Bei Bestrahlung mit Sonnenlicht verhalten sich Hautareale unterschiedlicher Pigmentierung unterschiedlich. Stark pigmentierte Hautareale, wie Altersflecken, bräunen ähnlich wie die normale wenig pigmentierte Haut. Nach einem Sonnenbad treten daher die stark pigmentierten Hautareale stärker hervor, als vor der Bestrahlung mit Sonnenlicht.

Aufgabe der Erfindung ist es daher, den Mangel sich verstärkender Hautpigmentierungsunterschiede bei Bestrahlung mit Sonnenlicht durch gezielte, verstärkte Pigmentierung/Bräunung der helleren Hautareale beizukommen und den Kontrast zwischen dunkler und heller pigmentierten Hautarealen zu vermindern. Damit wird dem Verbraucher ein optisch gleichmäßigeres Erscheinungsbild gegeben.

Die gezielte Pigmentierung der Haut ist auf verschiedene Weise möglich.

Unter gezielter Pigmentierung werden alle Verfahren verstanden, die eine Pigmentierung/Bräunung in den oberen Hautschichten auslösen. Dazu gehören nicht die Bedeckung der Haut mit Farbpigmenten, langläufig als Schminken bekannt oder die Applikation von Pigmenten in die Lederhaut (Corium) (Tätowieren).

Bei der "Direkt-Pigmentierung", die mit der Sonnenbestrahlung einsetzt, werden vorwiegend farblose Melanin-Vorstufen durch UVA-Strahlung zu dunkel gefärbten Melanin oxidiert. Da diese Oxidierung reversibel ist, führt sie zu einer nur kurz anhaltenden Hautbräunung. UVAbedingte Pigmentierungen grenzen sich gegenüber der UVB-vermittelten Bräunung (verzögerte Bräunung, Delayed Tanning, DT) durch ihre zeitliche Kinetik ab und werden als IPD (Immediate Pigment Darkening) und PPD (Persistent Pigment Darkening bezeichnet. IPD und PPD treten nach Bestrahlung deutlich vor der verzögerten Bäunung DT auf. Sowohl IPD als auch PPB können zur Bewertung der UVA-Schutzleistung eines Lichtschutzproduktes herangezogen werden.

Die menschliche Haut lässt sich jedoch auch künstlich durch orale Einnahme von Carotinoiden bräunen, was

jedoch zu unnatürlich wirkenden Orangefarbtönen der Haut führen kann.

Weitaus beliebter jedoch ist die künstliche Bräunung der Haut, welche sich durch Auftragen von sogenannten Selbstbräunern erzielen lässt. Diese Verbindungen weisen als chemisches Strukturmerkmal Keto- bzw. Aldehydgruppen in Nachbarschaft zu Alkoholfunktionen auf. Diese Ketole bzw. Aldole gehören überwiegend zur Substanzklasse der Zucker. Der am häufigsten verwendete Selbstbräuner ist das 1,3-Dihydroxyaceton. Die Verbindungen können mit den Proteinen und Aminosäuren der Hornschicht der Haut im Sinne einer Maillard-Reaktion umgesetzt werden, wobei über einen noch nicht vollständig aufgeklärten Reaktionsweg Polymerisate entstehen, die der Haut einen bräunlichen Farbton verleihen. Diese Reaktion ist nach etwa 4 bis 6 Stunden abgeschlossen; die so erzielte Bräune ist nicht abwaschbar und wird erst mit der normalen Hautabschuppung entfernt.

Die durch die Bestrahlung mit natürlichem Sonnenlicht auftretende Kontrastverstärkung zwischen helleren und dunkleren Hautarealen wird jedoch durch die gezielte Pigmentierung nicht entscheidend verringert.

Nicht vorhersehbar war, das die Verwendung von zwei Sonnenschutzmitteln, wobei ein Sonnenschutzmittel, welches einen niedrigen Lichtschutzfaktor aufweist (im folgenden als Basisschutz bezeichnet) und als Grundierung des gesamten zu schützenden Hautareals aufgetragen wird und ein weiteres Sonnenschutzmittel, welches einen hohen Lichtschutzfaktor aufweist (im folgenden als Fleckschutz bezeichnet) und selektiv auf die stark pigmentierten Hautareale aufgetragen wird, bei UV-Bestrahlung zu einer Kontrastverringerung zwischen stark pigmentierten und weniger pigmentierten Hautbereichen führt.

Hierbei handelt es sich nicht um eine ursächliche Therapie der Hyperpigmentierung, sondern nur um eine Kaschierung der Symptomatik.

Im Sinne der Erfindung ist als Sonnenschutzmittel eine topisch anzuwendende Zubereitung mit einem Gehalt an wenigstens einem Lichtschutzfilter zu verstehen, die die Haut vor einer übermäßigen Schädigung durch UV-Strahlung schützt. Dabei ist mit UV-Strahlung eine elektromagnetische Strahlung im Bereich von 290 nm bis 400 nm zu verstehen. Die UV-Strahlung kann natürlichem oder künstlichen Ursprungs sein.

Um zu guten Effekten zu kommen, müssen die Schutzleistungen zwischen dem Basisschutz und dem Fleckschutz bestimmte Kriterien erfüllen:

a) Die Schutzleistung, das heißt der Lichtschutzfaktor (Abkürzung LSF bzw. SPF) der beiden Sonnenschutzmittel muss sich um mindestens 10, bevorzugt mindestens 15, besonders bevorzugt um mindestens 20 LSF-Einheiten unterscheiden.

b) Die Schutzleistung des Basisschutzes sollte einen LSF von mindestens 5, bevorzugt mindestens 8, besonders bevorzugt mindestens 10 aufweisen, jedoch nicht mehr als LSF 30 betragen.

c) Das Verhältnis der Lichtschutzfaktoren (LSF) zwischen dem Fleckschutz und dem Basisschutz ($LFS_{Fleckschutz}/LSF_{Basisschutz}$) sollte bei mindestens 1.5, bevorzugt mindestens 2, besonders bevorzugt mindestens 2,5 liegen.

Die Bestimmung des LSF erfolgt gemäß den Richtlinien «International Sun Protection Factor Test Method, 2003» der COLIPA (Comité de Liaison des Associations Européennes de l'Industrie de la Parfumerie, des Produits Cosmétiques et de Toilette).

Ferner hatte sich gezeigt, dass sowohl zum Basisschutz wie auch zum Fleckschutz der UVA-Schutz eine wichtige Rolle spielt. Der UVA-Schutz wird in Form der UVA-Balance beschrieben. Die UVA-Balance beschreibt das Verhältnis zwischen der UVB- und der UVA-Schutzleistung eines Produktes, wobei die UVA-Balance, auch UV-Balance genannt, ist definiert als:

$$\text{UVA-Balance} = \frac{PPD_{\textit{in vitro}} - 1}{SPF_{\textit{in vivo}} - 1} \times 100$$

und wird detailliert in DIN 67502 beschrieben. Der PPD-Wert wird üblicherweise *in vitro* bestimmt, beispielsweise wie bei Wendel et al. beschrieben (V. Wendel et al., 2003, The influence of pre-irradiation on the predictability of in vivo UVA protection with a new in vitro method, Photodermatol Photoimmunol Photomed, 19:93-97). Hilfsweise kann der PPD-Wert alternativ auch *in vivo* bestimmt werden.

Der Wert für den SPF (Sun Protection Factor, auch "LSF" für Lichtschutzfaktor genannt) wird gemäß COLIPA *in vivo* bestimmt.

Der UVA-Schutz sollte hierbei der Gestalt sein, dass

d) die UVA-Balance des Fleckschutzes bei mindestens 20, bevorzugt mindestens 30, besonders bevorzugt bei mindestens 40 liegt

e) die UVA-Balance des Basisschutzes bei mindestens 15, bevorzugt mindestens 25, besonders bevorzugt bei mindestens 35 liegt.

Die Applikation des Basisschutzes erfolgt in üblicher Weise wie beim Auftragen einer Creme, z. B. durch Verreibung einer adäquaten Menge des Sonnenschutzmittels mit der Hand auf dem Applikationsbereich.

Die selektive, randscharfe Applikation des Fleckschutzes kann erfindungsgemäß mit Hilfe einer Tube mit einer spitzen und/oder kleinen Ausgabeöffnung, einen Pinsel, einem Wattestäbchen bzw. über einen geeigneten Stiftapplikator erfolgen.

Wesentlicher Bestandteil der vorliegenden Erfindung ist die Verwendung der Kombination aus dem Basisschutz und dem Fleckschutz. Sie erlaubt, die gesamte Haut ausreichend zu schützen und dabei hyperpigmentierte Areale weniger Stark in Erscheinung treten bzw. gänzlich verschwinden zu lassen.

Werden die in den zuvor genannten Punkten a) bis e) beschriebenen Randbedingungen eingehalten, wird mit dem erfinderischen Verfahren eine hervorragende Angleichung der Färbung von normaler Haut an stark pigmentierte Haut erreicht.

Durch mehrfache Anwendung des erfindungsgemäßen Verfahrens wird im Gegensatz zum herkömmlichen Anwendung nur eines Sonnenschutzmittels für alle Hautbereiche eine gleichmäßige (fleckenfreie) Tönung der hellen Hautpartien erreicht und der Kontrast zu dunkleren Hautpartien (z. B. zu Sommersprossen, Altersflecken) wird deutlich, mit bloßem Auge sichtbar, verringert.

Bevorzugt werden die erfindungsgemäßen Zubereitungen mindestens einmal, besonders bevorzugt zweimal täglich auf die zu bräunenden Hautpartien aufgetragen.

Die Zubereitungen, mit denen das erfinderische Verfahren ausgeführt werden kann, können in verschiedenen Formen vorliegen und eingesetzt werden. So können sie z. B. eine Emulsion vom Typ Öl-in-Wasser (O/W) oder eine multiple Emulsion, beispielsweise vom Typ Wasser-in-Öl-in-Wasser (W/O/W). Auch emulgatorfreie Formulierungen wie Hydrodipsersionen, Hydrogele oder eine Pickering-Emulsion sind vorteilhafte Ausführungsformen. In allen Zubereitungsformen ist jedoch ein Gehalt an Lichtschutzfiltern vorhanden.

Die Konsistenz der Formulierungen kann von pastösen Formulierungen über fließfähige Formulierungen bis hin zu dünnflüssigen, sprühbaren Produkten reichen. Dementsprechend können Cremes, Lotionen, Stifte oder Sprays formuliert werden.

Zur Anwendung werden die erfindungsgemäßen kosmetischen Zubereitungen in der für Kosmetika und Dermatika üblichen Weise auf die Haut in ausreichender Menge aufgebracht, wobei der Basisschutz vollflächig und der Fleckschutz selektiv appliziert wird.

Durch das erfinderische Verfahren lässt sich nicht nur eine gleichmäßige Hautfärbung erreichen, es lassen sich insbesondere auch von Natur aus oder durch krankhafte Veränderung unterschiedlich gefärbte Hautbereiche gleichmäßig einfärben.

Ein Vorteil der des erfindungsgemäßen Verfahrens ist der selektiv stärkere UV-Schutz der besonders ‚anfälligen' stark pigmentierten Bereiche.

**Lichtschutzfilter**

Dementsprechend enthalten die Zubereitungen im Sinne der vorliegenden Erfindung vorzugsweise mindestens eine UV-A- und/oder UV-B-Filtersubstanz. Die Formulierungen können, obgleich nicht notwendig, gegebenenfalls auch ein oder mehrere organische und/oder anorganische Pigmente als UV-Filtersubstanzen enthalten, welche in der Wasser- und/oder der Ölphase vorliegen können.

Bevorzugte anorganische Pigmente sind Metalloxide und/oder andere in Wasser schwerlösliche oder unlösliche Metallverbindungen, insbesondere Oxide des Titans ($TiO_2$), Zinks (ZnO), Eisens (z. B. $Fe_2O_3$), Zirkoniums ($ZrO_2$), Siliciums ($SiO_2$), Mangans (z. B. MnO), Aluminiums ($Al_2O_3$), Cers (z. B. $Ce_2O_3$), Mischoxide der entsprechenden Metalle sowie Abmischungen aus solchen Oxiden, sowie das Sulfat des Bariums ($BaSO_4$).

Die Titandioxid- Pigmente können sowohl in der Kristallmodifikation Rutil als auch Anatas vorliegen und können im Sinne der vorliegenden Erfindung vorteilhaft oberflächlich behandelt ("gecoatet") sein, wobei beispielsweise ein hydrophiler, amphiphiler oder hydrophober Charakter gebildet werden bzw. erhalten bleiben soll. Diese Oberflächenbehandlung kann darin bestehen, dass die Pigmente nach an sich bekannten Verfahren mit einer dünnen hydrophilen und/oder hydrophoben anorganischen und/oder organischen Schicht versehen werden. Die verschiedenen Oberflächenbeschichtung können im Sinne der vorliegenden Erfindung auch Wasser enthalten.

Anorganische Oberflächenbeschichtungen im Sinne der vorliegenden Erfindung können bestehen aus Aluminiumoxid ($Al_2O_3$), Aluminiumhydroxid $Al(OH)_3$, bzw. Aluminiumoxidhydrat (auch: Alumina, CAS-Nr.: 1333-84-2), Natri-

umhexametaphosphat $(NaPO_3)_6$, Natriummetaphosphat $(NaPO_3)_n$, Siliciumdioxid ($SiO_2$) (auch: Silica, CAS-Nr.: 7631-86-9), Zirkoniumoxid ($ZrO_2$) oder Eisenoxid ($Fe_2O_3$). Diese anorganischen Oberflächenbeschichtungen können allein, in Kombination und/oder in Kombination mit organischen Beschichtungsmaterialien vorkommen.

Hierzu werden Oxide, Oxidhydrate oder Phosphate beispielsweise der Elemente Al, Si, Zr in dichten Schichten auf die Pigmentoberfläche aufgefällt.

Die anorganische Nachbehandlung geschieht im allgemeinen in einer wässrigen Suspension des Pigmentes durch Zugabe löslicher Nachbehandlungschemikalien, wie z.B. Aluminiumsulfat, und anschließende Ausfällung des im neutralen Bereich schwerlöslichen Hydroxides durch gezielte Einstellung des pH-Wertes mit Natronlauge.

Nach der anorganischen Nachbehandlung werden die gecoateten Pigmente durch Filtration aus der Suspension abgetrennt und sorgfältig gewaschen, um die gelösten Salze zu entfernen, anschließend werden die isolierten Pigmente getrocknet.

Besonders bevorzugt im Sinne dieser Erfindung sind Titandioxide, auf die Aluminiumhydroxid auf die Oberfläche aufgebracht worden ist, wie z. B. die von Sun Chemical erhältlichen Titandioxid Typen C47-051 und C47- 5175. Weiterhin bevorzugte Pigmente sind Titandioxide, die mit Aluminium- und / oder Siliziumoxiden gecoated sind, wie z. B. von der Firma Krosnos Titan: Kronos 1071 und 1075 oder von der Firma Kingfisher: A310.03 Tudor Aspen.

Organische Oberflächenbeschichtungen im Sinne der vorliegenden Erfindung können bestehen aus pflanzlichem oder tierischem Aluminiumstearat, pflanzlicher oder tierischer Stearinsäure, Laurinsäure, Dimethylpolysiloxan (auch: Dimethicone), Methylpolysiloxan (Methicone), Simethicone (einem Gemisch aus Dimethylpolysiloxan mit einer durchschnittlichen Kettenlänge von 200 bis 350 Dimethylsiloxan-Einheiten und Silicagel) oder Alginsäure. Diese organischen Oberflächenbeschichtungen können allein, in Kombination und/oder in Kombination mit anorganischen Beschichtungsmaterialien vorkommen.

Beschriebene beschichtete und unbeschichtete Titandioxide können im Sinne vorliegender Erfindung auch in Form commerziell erhältlicher öliger oder wäßriger Vordispersionen zur Anwendung kommen. Diesen Vordispersionen können vorteilhaft Dispergierhilfmittel und/oder Solubilisationsvermittler zugesetzt sein.

Die erfindungsgemäßen Titandioxide zeichnen sich durch eine Primärpartikelgröße zwischen 10 nm bis 150 nm aus.

| **Handelsname** | **Coating** | **zusätzliche Bestandteile der Vordispersion** | **Hersteller** |
|---|---|---|---|
| MT-100TV | Aluminiumhydroxid Stearinsäure | - | Tayca Corporation |
| MT-100Z | Aluminiumhydroxid Stearinsäure | - | Tayca Corporation |
| MT-100F | Stearinsäure Eisenoxid | - | Tayca Corporation |
| MT-500SAS | Alumina, Silica Silikon | - | Tayca Corporation |
| MT-100AQ | Silica Aluminiumhydroxid Alginsäure | - | Tayca Corporation |
| Eusolex T-2000 | Alumina Simethicone | - | Merck KGaA |
| Eosolex TS | Alumina, Stearinsäure - | | Merck KGaA |
| Titandioxid P25 | None | - | Degussa |
| Titandioxid T805 (Uvinul $TiO_2$) | Octyltrimethylsilan | - | Degussa |
| UV-Titan X170 | Alumina Dimethicone | - | Kemira |
| UV-Titan X161 | Alumina, Silica Stearinsäure | - | Kemira |
| Tioveil AQ 10PG | Alumina Silica | Wasser Propylenglycol | Solaveil Uniquema |
| Mirasun TiW 60 | Alumina Silica | Wasser | Rhone-Poulenc |

Im Sinne der vorliegenden Erfindung sind besonders bevorzugte Titandioxide das MT-100 Z und MT-100 TV von Tayca Corporation, Eusolex T-2000 und Eusolex TS von Merck und das Titandioxid T 805 von Degussa.

Zinkoxide können im Sinne der vorliegenden Erfindung auch in Form kommerziell erhältlicher öliger oder wäßriger Vordispersionen zur Anwendung kommen. Erfindungsgemäß geeignete Zinkoxidpartikel und Vordispersionen

von Zinkoxidpartikeln zeichnen sich durch eine Primärpartikelgröße von < 300 nm aus und sind unter folgenden Handelsbezeichnungen bei den aufgeführten Firmen erhältlich:

| Handelsname | Coating | Hersteller |
|---|---|---|
| Z- Cote HP1 | 2% Dimethicone | BASF |
| Z- Cote | / | BASF |
| ZnO NDM | 5% Dimethicone | H&R |
| MZ 707M | 7% Dimethicone | M. Tayca Corp. |
| Nanox 500 | / | Elementis |
| ZnO Neutral | / | H&R |

Besonderes bevorzugte Zinkoxide im Sinne der Erfindung sind das Z-Cote HP1 von der Firma BASF und das Zinkoxid NDM von der Firma Haarmann & Reimer.

Die Gesamtmenge an einem oder mehreren anorganischen Pigmenten in der fertigen kosmetischen Zubereitung wird vorteilhaft aus dem Bereich 0,1 Gew.-% bis 25 Gew.-% gewählt, vorzugsweise 0,5 Gew.-% bis 18 Gew.-%.

Vorteilhaftes organisches Pigment im Sinne der vorliegenden Erfindung ist das 2,2'-Methylen-bis-(6-(2H-benzotriazol-2-yl)-4-(1,1,3,3-tetramethylbutyl)-phenol) [INCI: Bisoctyltriazol], welches durch die chemische Strukturformel

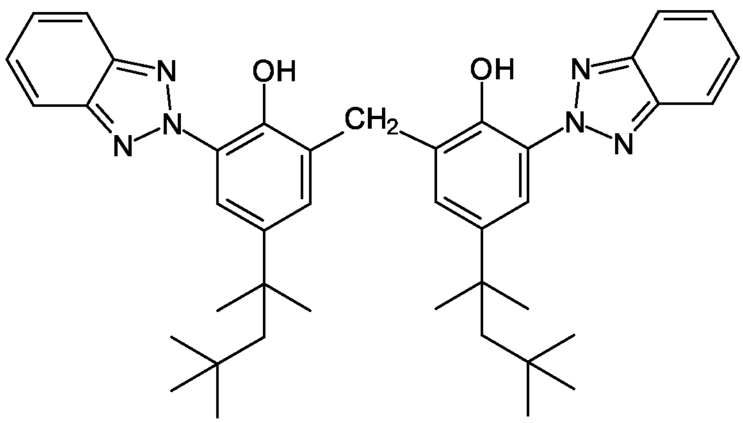

gekennzeichnet ist und unter der Handelsbezeichnung Tinosorb® M bei der CIBA-Chemikalien GmbH erhältlich ist.

Vorteilhafte UV-A-Filtersubstanzen im Sinne der vorliegenden Erfindung sind Dibenzoylmethanderivate, insbesondere das 4-(tert.-Butyl)-4'-methoxydibenzoylmethan (CAS-Nr. 70356-09-1), welches von Givaudan unter der Marke Parsol® 1789 und von Merck unter der Handelsbezeichnung Eusolex® 9020 verkauft wird.

Weitere vorteilhafte UV-A-Filtersubstanzen sind die Phenylen-1,4-bis-(2-benzimidazyl)-3,3'-5,5'-tetrasulfonsäure

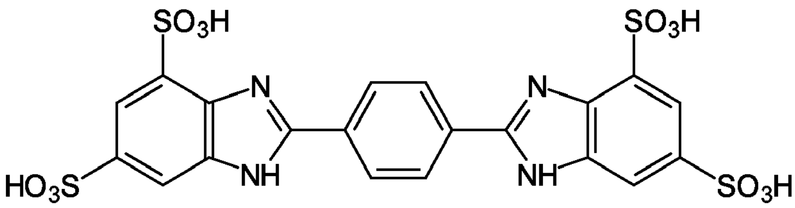

und ihre Salze, besonders die entsprechenden Natrium-, Kalium- oder Triethanolammonium-Salze, insbesondere das

Phenylen-1,4-bis-(2-benzimidazyl)-3,3'-5,5'-tetrasulfonsäure-bis-natriumsalz

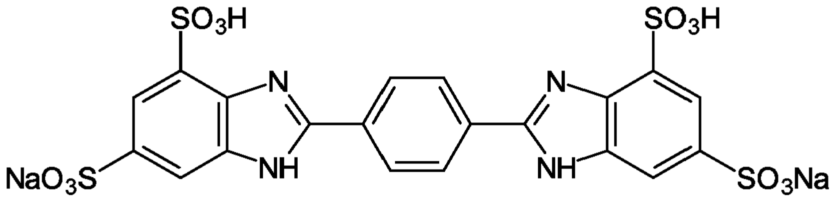

mit der INCI-Bezeichnung Bisimidazylate, welches beispielsweise unter der Handelsbezeichnung Neo Heliopan AP bei Haarmann & Reimer erhältlich ist.

Ferner vorteilhaft sind das 1,4-di(2-oxo-10-Sulfo-3-bornylidenmethyl)-Benzol und dessen Salze (besonders die entprechenden 10-Sulfato-verbindungen, insbesondere das entsprechende Natrium-, Kalium- oder Triethanolammonium-Salz), das auch als Benzol-1,4-di(2-oxo-3-bornyliden-methyl-10-sulfonsäure) bezeichnet wird und sich durch die folgende Struktur auszeichnet:

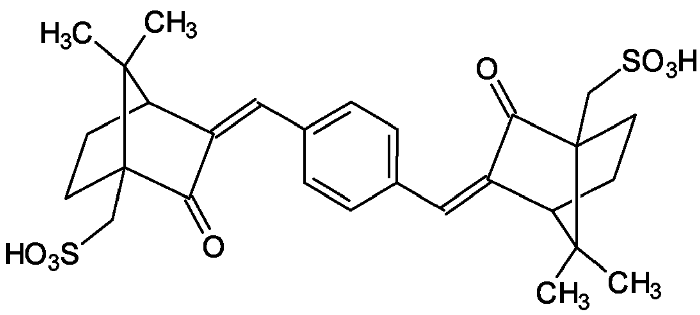

Weitere vorteilhafte UV-A-Filtersubstanzen sind Hydroxybenzophenone, die sich durch die folgende Strukturformel auszeichnen:

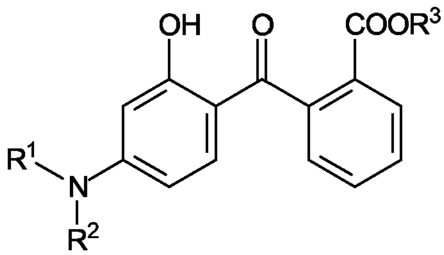

worin

- $R^1$ und $R^2$ unabhängig voneinander Wasserstoff, $C_1$-$C_{20}$-Alkyl, $C_3$-$C_{10}$-Cycloalkyl oder $C_{3-}C_{10}$-Cycloalkenyl bedeuten, wobei die Substituenten $R^1$ und $R^2$ gemeinsam mit dem Stickstoffatom, an das sie gebunden sind, einen 5- oder 6-Ring bilden können und
- $R^3$ einen $C_1$-$C_{20}$-Alkyl Rest bedeutet.

Ein besonders vorteilhaftes Hydroxybenzophenon im Sinne der vorliegenden Erfindung ist der 2-(4'-Diethylamino-2'-hydooybenzoyl)-benzoesäureheoylester (auch: Aminobenzophenon), welcher sich durch folgende Struktur auszeichnet:

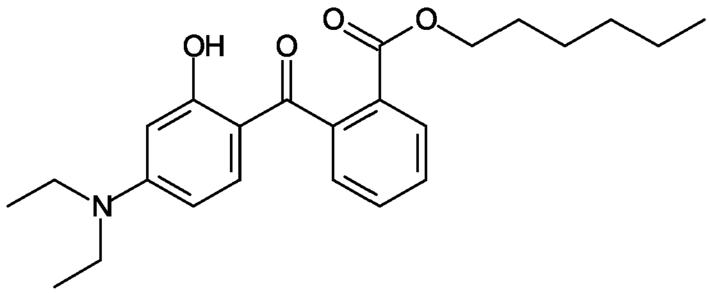

und unter dem Handelsnamen Uvinul A Plus bei der Fa. BASF erhältlich ist.

Vorteilhafte UV-Filtersubstanzen im Sinne der vorliegenden Erfindung sind ferner sogenannte Breitbandfilter, d.h. Filtersubstanzen, die sowohl UV-A- als auch UV-B-Strahlung absorbieren.

Vorteilhafte Breitbandfilter oder UV-B-Filtersubstanzen sind beispielsweise Bis-Resorcinyltriazinderivate mit der folgenden Struktur:

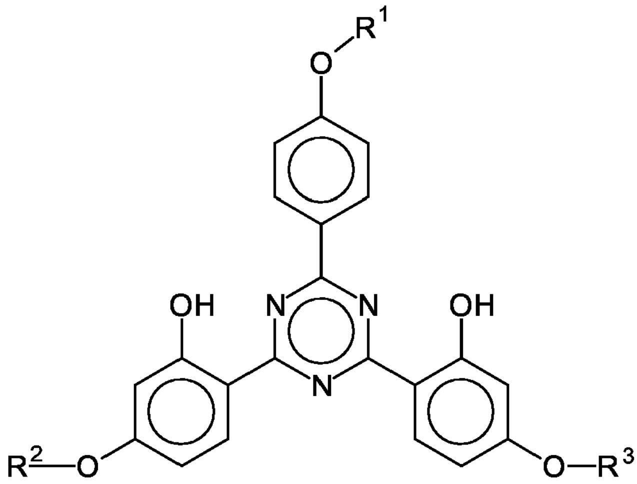

wobei $R^1$, $R^2$ und $R^3$ unabhängig voneinander gewählt werden aus der Gruppe der verzweigten und unverzweigten Alkylgruppen mit 1 bis 10 Kohlenstoffatomen bzw. ein einzelnes Wasserstoffatom darstellen. Insbesondere bevorzugt sind das 2,4-Bis-{[4-(2-Ethyl-hexyloxy)-2-hydroxy]-phenyl}-6-(4-methoxyphenyl)-1,3,5-triazin (INCI: Aniso Triazin), welches unter der Handelsbezeichnung Tinosorb® S bei der CIBA-Chemikalien GmbH erhältlich ist.

Besonders vorteilhafte Zubereitungen im Sinne der vorliegenden Erfindung, die sich durch einen hohen bzw. sehr hohen UV-A-Schutz auszeichnen, enthalten bevorzugt mehrere UV-A-und/oder Breitbandfilter, insbesondere Dibenzoylmethanderivate [beispielsweise das 4-(tert.-Butyl)-4'-methoxydibenzoylmethan], Benzotriazolderivate [beispielsweise das 2,2'-Methylen-bis-(6-(2H-benzotriazol-2-yl)-4-(1,1,3,3-tetramethylbutyl)-phenol)], Phenylen-1,4-bis-(2-benzimidazyl)-3,3'-5,5'-tetrasulfonsäure und/oder ihre Salze, das 1,4-di(2-oxo-10-Sulfo-3-bornyliden-methyl)-Benzol und/oder dessen Salze und/oder das 2,4-Bis-{[4-(2-Ethyl-hexyloxy)-2-hydroxy]-phenyl}-6-(4-methoxyphenyl)-1,3,5-triazin, jeweils einzeln oder in beliebigen Kombinationen miteinander.

Auch andere UV-Filtersubstanzen, welche das Strukturmotiv

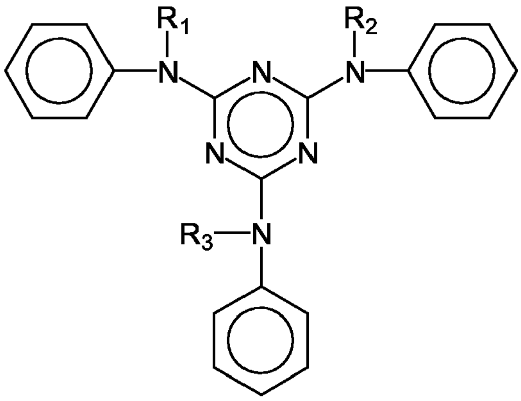

aufweisen, sind vorteilhafte UV-Filtersubstanzen im Sinne der vorliegenden Erfindung, beispielsweise die in der Europäischen Offenlegungsschrift EP 570 838 A1 beschriebenen s-Triazinderivate, deren chemische Struktur durch die generische Formel

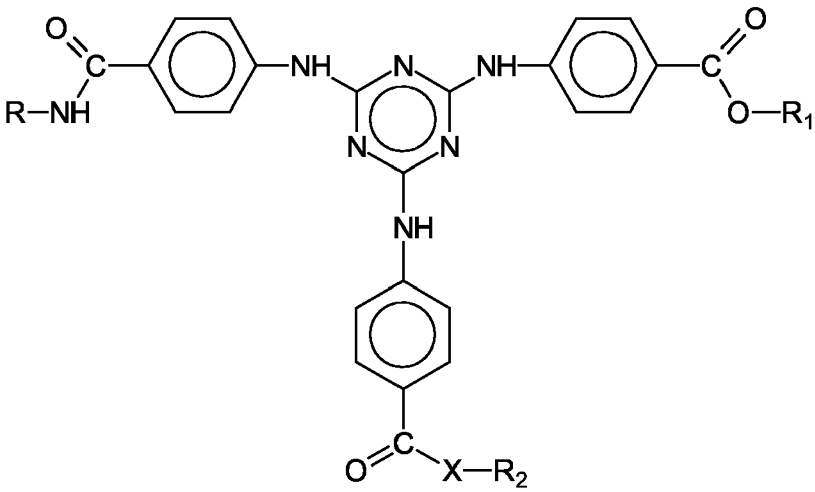

wiedergegeben wird, wobei

R einen verzweigten oder unverzweigten $C_1$-$C_{18}$-Alkylrest, einen $C_5$-$C_{12}$-Cycloalkylrest, gegebenenfalls substituiert mit einer oder mehreren $C_1$-$C_4$- Alkylgruppen, darstellt,

X ein Sauerstoffatom oder eine NH-Gruppe darstellt,

$R_1$ einen verzweigten oder unverzweigten $C_1$-$C_{18}$-Alkylrest, einen $C_5$-$C_{12}$-Cycloalkylrest, gegebenenfalls substituiert mit einer oder mehreren $C_1$-$C_4$- Alkylgruppen, oder ein Wasserstoffatom, ein Alkalimetallatom, eine Ammoniumgruppe oder eine Gruppe der Formel

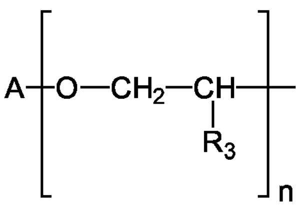

bedeutet, in welcher

A einen verzweigten oder unverzweigten $C_1$-$C_{18}$-Alkylrest, einen $C_5$-$C_{12}$-Cycloalkyl- oder Arylrest darstellt, gegebenenfalls substituiert mit einer oder mehreren $C_1$-$C_4$-Alkylgruppen,

$R_3$ ein Wasserstoffatom oder eine Methylgruppe darstellt,

n eine Zahl von 1 bis 10 darstellt,

$R_2$ einen verzweigten oder unverzweigten $C_1$-$C_{18}$-Alkylrest, einen $C_5$-$C_{12}$-Cycloalkylrest, gegebenenfalls substituiert mit einer oder mehreren $C_1$-$C_4$- Alkylgruppen, darstellt, wenn X die NH-Gruppe darstellt, und einen verzweigten oder unverzweigten $C_1$-$C_{18}$-Alkylrest, einen $C_5$-$C_{12}$-Cycloalkylrest, gegebenenfalls substituiert mit einer oder mehreren $C_1$-$C_4$- Alkylgruppen, oder ein Wasserstoffatom, ein Alkalimetallatom, eine Ammoniumgruppe oder eine Gruppe der Formel

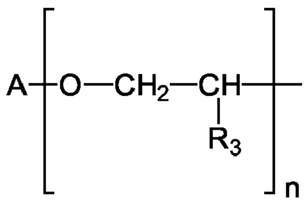

bedeutet, in welcher

A einen verzweigten oder unverzweigten $C_1$-$C_{18}$-Alkylrest, einen $C_5$-$C_{12}$-Cycloalkyl- oder Arylrest darstellt, gegebenenfalls substituiert mit einer oder mehreren $C_1$-$C_4$-Alkylgruppen,

$R_3$ ein Wasserstoffatom oder eine Methylgruppe darstellt,

n eine Zahl von 1 bis 10 darstellt,

wenn X ein Sauerstoffatom darstellt.

Besonders bevorzugte UV-Filtersubstanz im Sinne der vorliegenden Erfindung ist ferner ein unsymmetrisch substituiertes s-Triazin, dessen chemische Struktur durch die Formel

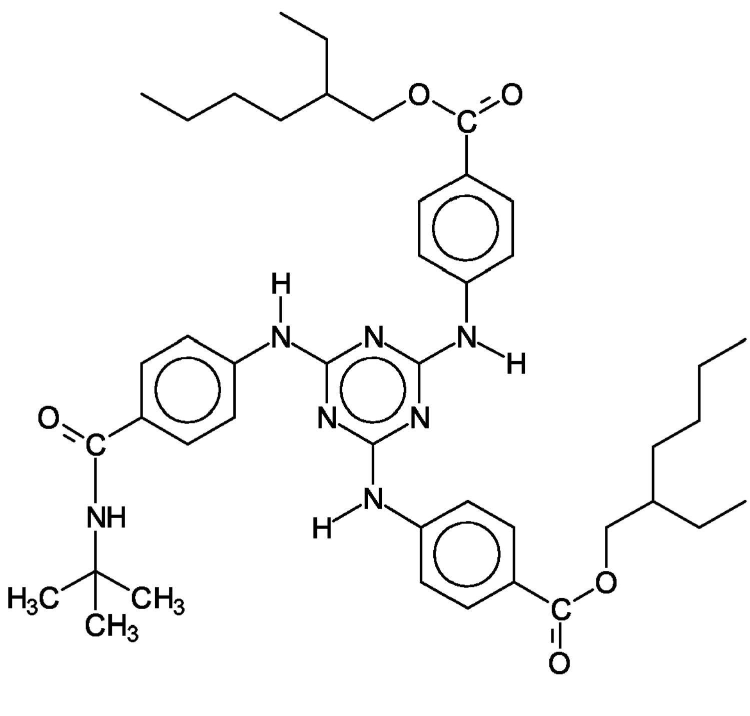

wiedergegeben wird, welches im Folgenden auch als Dioctylbutylamidotriazon (INCI: Dioctylbutamidotriazone) bezeichnet wird und unter der Handelsbezeichnung UVASORB HEB bei Sigma 3V erhältlich ist.

Vorteilhaft sind ferner Benzoxazol-Derivate, wie z. B. das 2,4-bis-[5-1 (dimethylpropyl)benzoxazol-2-yl-(4-phenyl)-imino]-6-(2-ethylhexyl)-imino-1,3,5-triazin mit der CAS Nr. 288254-16-0, welches bei 3V Sigma unter der Handelsbezeichnung Uvasorb® K2A erhältlich ist;

Vorteilhaft im Sinne der vorliegenden Erfindung ist auch ein symmetrisch substituiertes s-Triazin, das 4,4',4''-(1,3,5-Triazin-2,4,6-triyltriimino)-tris-benzoësäure-tris(2-ethylhexylester), synonym: 2,4,6-Tris-[anilino-(p-carbo-2'-ethyl-1'-hexyloxy)]-1,3,5-triazin (INCI: Octyl Triazone), welches von der BASF Aktiengesellschaft unter der Warenbezeichnung UVINUL® T 150 vertrieben wird.

Auch in der Europäischen Offenlegungsschrift 775 698 werden bevorzugt einzusetzende Bis-Resorcinyltriazinderivate beschrieben, deren chemische Struktur durch die generische Formel

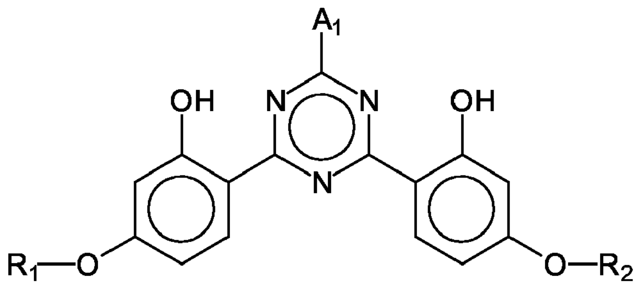

wiedergegeben wird, wobei $R_1$, $R_2$ und $A_1$ verschiedenste organische Reste repräsentieren.

Vorteilhaft im Sinne der vorliegenden Erfindung sind ferner das 2,4-Bis-{[4-(3-sulfonato)-2-hydroxy-propyloxy)-2-hydroxy]-phenyl}-6-(4-methoxyphenyl)-1,3,5-triazin Natriumsalz, das 2,4-Bis-{[4-(3-(2-Propyloxy)-2-hydroxy-propyloxy)-2-hydroxy]-phenyl}-6-(4-methoxyphenyl)-1,3,5-triazin, das 2,4-Bis-{[4-(2-ethyl-hexyloxy)-2-hydroxy]-phenyl}-6-[4-(2-methoxyethyl-carboxyl)-phenylamino]-1,3,5-triazin, das 2,4-Bis-{[4-(3-(2-propyloxy)-2-hydroxy-propyloxy)-2-hydroxy]-phenyl}-6-[4-(2-ethyl-carboxyl)-phenylamino]-1,3,5-triazin, das 2,4-Bis-{[4-(2-ethyl-hexyloxy)-2-hydroxy]-phenyl}-6-(1-methyl-pyrrol-2-yl)-1,3,5-triazin, das 2,4-Bis-{[4-tris(trimethylsiloxy-silylpropyloxy)-2-hydroxy]-phenyl}-6-(4-methoxyphenyl)-1,3,5-triazin, das 2,4-Bis-{[4-(2''-methyl-propenyloxy)-2-hydroxy]-phenyl}-6-(4-methoxyphenyl)-1,3,5-triazin und das 2,4-Bis-{[4-(1',1',1',3',5',5',5'-Heptamethylsiloxy-2''-methyl-propyloxy)-2-hydroxy]-phenyl}-6-(4-methoxyphenyl)-1,3,5-triazin.

Ein vorteilhafter Breitbandfilter im Sinne der vorliegenden Erfindung ist das 2,2'-Methylen-bis-(6-(2H-benzotriazol-2-yl)-4-(1,1,3,3-tetramethylbutyl)-phenol), welches durch die chemische Strukturformel

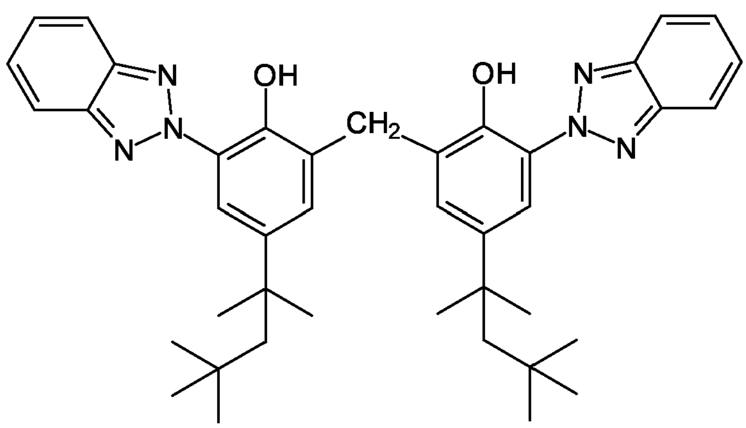

gekennzeichnet ist und unter der Handelsbezeichnung Tinosorb® M bei der CIBA-Chemikalien GmbH erhältlich ist.

Vorteilhafter Breitbandfilter im Sinne der vorliegenden Erfindung ist ferner das 2-(2H-benzotriazol-2-yl)-4-methyl-6-[2-methyl-3-[1,3,3,3-tetramethyl-1-[(trimethylsilyl)oxy]disiloxanyl]propyl]-phenol (CAS-Nr.: 155633-54-8) mit der INCI-Bezeichnung Drometrizole Trisiloxane, welches durch die chemische Strukturformel

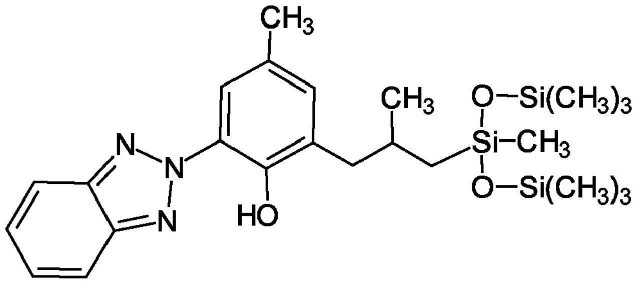

gekennzeichnet ist.

Die UV-B- und/oder Breitband-Filter können öllöslich oder wasserlöslich sein. Vorteilhafte öllösliche UV-B- und/oder Breitband-Filtersubstanzen sind z. B.:

- ■ 3-Benzylidencampher-Derivate, vorzugsweise 3-(4-Methylbenzyliden)campher, 3-Benzylidencampher;
- ■ 4-Aminobenzoesäure-Derivate, vorzugsweise 4-(Dimethylamino)-benzoesäure(2-ethylhexyl)ester, 4-(Dimethylamino)benzoesäureamylester;
- ■ 2,4,6-Trianilino-(p-carbo-2'-ethyl-1'-hexyloxy)-1,3,5-triazin;
- ■ Ester der Benzalmalonsäure, vorzugsweise 4-Methoxybenzalmalonsäuredi(2-ethylhexyl)-ester;
- ■ Ester der Zimtsäure, vorzugsweise 4-Methoxyzimtsäure(2-ethylhexyl)ester, 4-Methoxyzimtsäureisopentylester;
- ■ Derivate des Benzophenons, vorzugsweise 2-Hydroxy-4-methoxybenzophenon, 2-Hydroxy-4-methoxy-4'-methylbenzophenon, 2,2'-Dihydroxy-4-methoxybenzophenon
- ■ sowie an Polymere gebundene UV-Filter.

Vorteilhafte wasserlösliche UV-B- und/oder Breitband-Filtersubstanzen sind z. B.:

- ■ Salze der 2-Phenylbenzimidazol-5-sulfonsäure, wie ihr Natrium-, Kalium- oder ihr Triethanolammonium-Salz, sowie die Sulfonsäure selbst;
- ■ Sulfonsäure-Derivate des 3-Benzylidencamphers, wie z. B. 4-(2-Oxo-3-bornylidenmethyl)-benzolsulfonsäure, 2-Methyl-5-(2-oxo-3-bornylidenmethyl)sulfonsäure und deren Salze.

Besonders vorteilhafte bei Raumtemperatur flüssige UV-Filtersubstanzen im Sinne der vorliegenden Erfindung sind Homomenthylsalicylat (INCI: Homosalate), 2-Ethylhexyl-2-hydroxybenzoat (2-Ethylhexylsalicylat, Octylsalicylat, INCI: Octyl Salicylate), 4-Isopropylbenzylsalicylat und Ester der Zimtsäure, vorzugsweise 4-Methoxyzimtsäure(2-ethylhexyl)ester (2-Ethylhexyl-4-methoxycinnamat, INCI: Octyl Methoxycinnamate) und 4-Methoxyzimtsäureisopentylester (Isopentyl-4-methoxycinnamat, INCI: Isoamyl p-Methoxycinnamate), 3-(4-(2,2-bis Ethoxycarbonylvinyl)-phenoxy)propenyl)-methoxysiloxan/ Dimethylsiloxan - Copolymer (INCI: Dimethicodiethylbenzalmalonat) welches beispielsweise unter der Handelsbezeichnung Parsol® SLX bei Hoffmann La Roche erhältlich ist.

Eine weiterere erfindungsgemäß vorteilhaft zu verwendende Lichtschutzfiltersubstanz ist das Ethylhexyl-2-cyano-3,3-diphenylacrylat (Octocrylen), welches von BASF unter der Bezeichnung Uvinul® N 539 erhältlich ist und sich durch folgende Struktur auszeichnet:

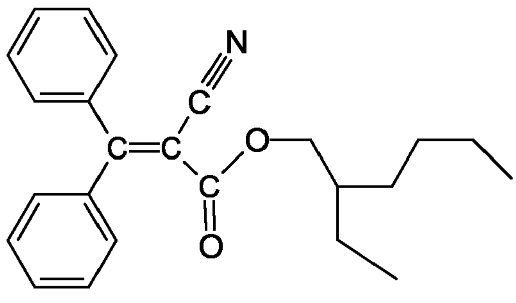

Es kann auch von erheblichem Vorteil sein, polymergebundene oder polymere UV-Filtersubstanzen in Zubereitungen gemäß der vorliegenden Erfindung zu verwenden, insbesondere solche, wie sie in der WO-A-92/20690 beschrieben werden.

Die Liste der genannten UV-Filter, die im Sinne der vorliegenden Erfindung eingesetzt werden können, soll selbstverständlich nicht limitierend sein.

Vorteilhaft enthalten die erfindungsgemäßen Zubereitungen die Substanzen, die UV-Strahlung im UV-A- und/ oder UV-B-Bereich absorbieren, in einer Gesamtmenge von z. B. 0,1 Gew.-% bis 30 Gew.-%, vorzugsweise 0,5 bis 20 Gew.-%, insbesondere 1,0 bis 15,0 Gew.-%, jeweils bezogen auf das Gesamtgewicht der Zubereitungen, um kosmetische Zubereitungen zur Verfügung zu stellen, die die Haut vor dem gesamten Bereich der ultravioletten Strahlung schützen.

**Selbstbräuner**

Die Zubereitungen gemäß der vorliegenden Erfindung können ferner vorteilhaft auch Selbstbräunungssubstanzen wie beispielsweise Dihydroxyacteon, Melaninderivate und Erythrulose enthalten, wobei diese Substanzen in Konzentrationen von 0,1 Gew.-% bis zu 8 Gew.-% bezogen auf das Gesamtgewicht der Zubereitung eingesetzt werden. Die erfindungsgemäßen Formulierungen können ferner besonders vorteilhaft neben Dihydroxyaceton auch Nussextrakte enthalten sowie weitere Substanzen, welche die Bräune erhalten oder erzeugen oder zusätzlich verstärken sollen. Insbesondere Vorteilhaft ist es die Selbstbräunungssubstanzen mit dem Basisschutz zu applizieren, da dadurch die helle Haut stärker gebräunt wird.

**Moisturizer**

Erfindungsgemäß vorteilhaft ist, wenn die Sonnenschutzmittel Substanzen beinhalten, die sich positiv auf die Hautbefeuchtung auswirken. Solche Substanzen oder Substanzgemnische werden als Moisturizer bezeichnet. Sie verleihen kosmetischen oder dermatologischen Zubereitungen die Eigenschaft, nach dem Auftragen bzw. Verteilen auf der Hautoberfläche die Feuchtigkeitsabgabe der Hornschicht (auch transepidermal water loss (TEWL) genannt) zu reduzieren und/oder die Hydratation der Hornschicht positiv zu beeinflussen.

Vorteilhaft im Sinne der vorliegenden Erfindung werden der oder die Moisturizer gewählt aus der Gruppe: Glycerin, Milchsäure und/oder Lactate, insbesondere Natriumlactat, Butylenglykol, Propylenglykol, Biosaccaride Gum-1, Glycine Soja, Ethylhexyloxyglycerin, Pyrrolidoncarbonsäure und Harnstoff. Ferner ist es insbesondere von Vorteil, polymere Moisturizer aus der Gruppe der wasserlöslichen, in Wasser quellbaren oder mit Hilfe von Wasser gelierbaren Polysaccharide zu verwenden. Insbesondere vorteilhaft sind beispielsweise Hyaluronsäure, Chitosan und ein fucosereiches Polysaccharid, welches in den Chemical Abstracts unter der Registraturnummer 178463-23-5 abgelegt und z. B. unter der Bezeichnung Fucogel®1000 von der Gesellschaft SOLABIA S.A. erhältlich ist. Moisturizer können vorteilhaft auch als Antifaltenwirkstoffe zur Prophylaxe und Behandlung kosmetischer oder dermatologischer Hautveränderungen, wie sie z. B. bei der Hautalterung auftreten, verwendet werden. Es ist besonders vorteilhaft im Sinne der vorliegenden Erfindung, Glycerin als Moisturizer zu verwenden, bevorzugt in einer Konzentration von 0,05 bis 30 Gew.-%, besonders bevorzugt 1 bis 10 Gew.-%, jeweils bezogen auf das Gesamtgewicht der Zubereitung.

**Wirkstoffe**

Medizinische topische Zusammensetzungen im Sinne der vorliegenden Erfindung enthalten in der Regel ein oder mehrere Wirkstoffe in kosmetisch wirksamer Konzentration.

So zeigte sich bei dem erfinderischen Verfahren überraschenderweise, dass die erfindungsgemäßen Sonnenschutzmittel sich auch ganz besonders eignen für Applikation von Wirkstoffen, die den Zustand der Haut positiv beeinflussen. So zeigte sich, dass Wirkstoffe zur positiven Beeinflussung der Altershaut, die die Entstehung von Falten oder auch bestehenden Falten vermindern.

Bevorzugte Wirkstoffe zur positiven Beeinflussung von Altershaut sind Biochinone, insbesondere Ubichinon Q10, Kreatin, Kreatinin, Carnitin, Biotin, Isoflavon, Cardiolipin, Liponsäure, Liponamid, Folsäure und ihre Derviate, Niacin und seine Derivate (insbesondere Niacinamid), Arctiin, Anti Freezing Proteine, Hopfen- und Hopfen-Malz-Extrakte. Auch fördernde Mittel der Restrukturierung des Bindegewebes, wie Isoflavonoide sowie Isoflavonoid-haltige Pflanzenextrakte wie z. B. Soja- und KleeExtrakte können in dem erfindungsgemäßen Verfahren sehr gut verwendet werden.

Auch zeigt sich, dass sich die für das erfindungsgemäße Verfahren verwendbaren Sonnenschutzmittel in besonderer Weise eignen, Wirkstoffe zur Unterstützung der Hautfunktionen bei trockener Haut, wie beispielsweise Vitamin C, Biotin, Carnitin, Kreatin, Propionsäure, Grüntee-Extrakte, Eucalyptusöl, Harnstoff und Mineralsalze wie z. B. NaCl, Meeresmineralien sowie Osmolyte wie z. B. Taurin, Inositol, Betain, quartäre Ammoniumverbindungen, zu applizieren. In ähnlicher Weise erwies sich die Applikation von Wirkstoffen zur Linderung bzw. positiven Beeinflussung von

irritativen Hautzuständen, sei es bei empfindlicher Haut im allgemeinen oder bei durch Noxen gereizter Haut (UV-Licht, Chemikalien), als vorteilhaft. Hier sind Wirkstoffe zu nennen wie Sericoside, verschiedene Extrakte des Süssholzes, Licochalcone, insbesondere Licochalcone A, Silymarin, Silyphos, Dexpanthenol, Inhibitoren des Prostaglandinstoffwechsels, insbesondere der Cyclooxygenase und des Leukotrienstoffwechsels, insbesondere der 5-Lipoxyaenase, aber auch des 5-Lipoxvgenase Inhibitor Proteins, FLAP.

Auch erwies sich ein Gehalt an Pigmentierungsmodulatoren als vorteilhaft. Hier sind insbesondere Modulatoren zu nennen, die die Pigmentierung der Haut vermindern und so zu einer kosmetisch gewünschten Aufhellung der Haut führen und/oder das Auftreten von Altersflecken und sonstigen lokalen Hyperpigmentierungen (insbesondere Melasma) reduzieren und/oder derartige Hyperpigmentierungen aufhellen. Der Einsatz von pigmentierungsmindernden Wirkstoffen erfolgt insbesondere in den Sonnenschutzmittel zum Fleckschutz. Beispielhaft seien als pigmentierungsmindernde Wirkstoffe erwähnt Tyrosinsulfat, Dioic acid (8-Hexadecen-1,16-dicarbonsäure sowie Liponsäure und Liponamid, verschiedene Extrakte des Süssholzes. Kojisäure, Hydrochinon, Arbutin, alpha-Arbutin, Deoxyarbutin, Fruchtsäuren, insbesondere Alpha-Hydroxy-Säuren (AHAs), Bearberry (Uvae ursi), Ursolsäure, Ascorbinsäure.

Grüntee-Extrakte, Aminoguanidin, Pyridoxamin.

Besonders vorteilhaft für den Einsatz in Sonnenschutzmitteln zum Basisschutz erwiesen sich Zubereitungen die Wirkstoffe, die eine verstärkte oder schnellere Bräunung der Haut herbeiführen (Advanced Glycation) Endproducts (AGE), Lipofuscine, NukleinsäureOligonukleotide, Dihydroxyaceton, Erythrulose, Purine und Pyrimidine, NO-freisetzende Substanzen, sei es mit oder ohne Einfluss von UV-Licht, enthalten.

**Mattierende und färbende Inhaltsstoffe**

Ferner kann in einer bevorzugten Ausführungsform der erfindungsgemäßen Formulierungen zusätzlich ein oder mehrere mattierende oder leicht überdeckende Inhaltsstoffe (Farbstoffe und/oder Pigmente) enthalten sein.

Ganz besonders vorteilhaft ist es als mattierende Inhaltsstoffe (Puderrohstoffe) Zuckerderivate aus der Gruppe der Stärken und/oder Stärkederivaten und/oder Cyclodextrine und/oder Cyclodextrinderivaten zu verwenden, wobei das Gewichtsverhältnis der Gesamtmenge an Stärke und/oder Stärkederivaten zur Gesamtmenge an Cyclodextrinen und/oder Cyclodextrinderivaten erfindungsgemäß vorteilhaft von 1:7 bis zu 15:1, bevorzugt von 1:1 bis 10:1 beträgt.

Es ist erfindungsgemäß vorteilhaft, wenn die Stärke und/oder Stärkederivate gewählt werden aus der Gruppe Distärkephosphat, Tapioka Stärke, Acetyl- bzw. Adipinsäure substituierte Stärke, Hydroxypropylierte Stärke, Aluminium- bzw. Natrium-Stärke, Stärke sustituiert mit N-Octenyl-Succinat, Maisstärke, 2-Hydropropylether modifizierte Stärke und Hydroxypropylstärke-Phosphatester und dergleichen, wobei Distärkephosphat (INCI: Distarch Phosphat) und Tapioka-Stärke (INCI: Tapioca Starch) erfindungsgemäß bevorzugt sind. Diese Stärken und Stärkederivate sind auch vorteilhaft, da sie zudem die sensorischen und kosmetischen Eigenschaften der Formulierungen weiter verbessern und beispielsweise ein samtiges oder seidiges Hautgefühl hervorrufen oder verstärken.

Es ist erfindungsgemäß von Vorteil, wenn die Cyclodextrine und/oder Cyclodextrinderivate gewählt werden aus der Gruppe $\alpha$-, $\beta$-, $\gamma$- und Hydroxypropyl-$\beta$- Cyclodextrin, wobei $\beta$- Cyclodextrin (INCI: Cyclodextrin) erfindungsgemäß bevorzugt ist.

Es ist erfindungsgemäß vorteilhaft, die erfindungsgemäßen Puderrohstoffe aus der Gruppe der Zuckerderivate in einer Gesamtmenge von 0,5 bis 10 Gewichts-%, bevorzugt in einer Menge von 2 bis 8 Gewichts % und ganz besonders bevorzugt in einer Menge von 3 bis 6 Gew.-%, jeweils bezogen auf das Gesamtgewicht der Zubereitung einzusetzen.

Unter den Zuckerderivaten ist des weiteren erfindungsgemäß vorteilhaft ist die Verwendung von Polysacchariden und -derivaten, wobei hier beispielsweise zu nennen wären Hyaluronsäure, Chitin und Chitosan, Chondroitinsulfate, Stärke und Stärkederivate, sowie die Verwendung von Cellulosederivaten, wie z.B. Methylcellulose, Carboxymethylcellulose, Hydroxyethylcellulose, Hydroxypropylmethylcellulose.

Weiterhin erfindungsgemäß vorteilhaft ist die Verwendung von Schichtsilikaten als mattierende Inhaltsstoffe.

Unter den Schichtsilikaten befinden sich natürlich vorkommende und synthetische Tonerden wie z. B. Montmorillonit, Bentonit, Hektorit, Laponit, Magnesiumaluminiumsilikate wie Veegum®. Diese können als solche oder in modifizierter Form als Verdicker verwendet werden wie z. B. Stearylalkonium Hektorite.

Weiterhin können vorteilhaft als mattierende Inhaltsstoffe auch Kieselsäuregele verwendet werden.

Auch ist es vorteilhaft, Antitranspirant-Salze (z. B. saure Aluminium- und/oder Aluminium/Zirkoniumsalze wie Aluminiumchlorhydrat und/oder Aluminium/ Zirkoniumchlorhydrat) und Elektrolyte zu verwenden. Antitranspirant-Salze können dabei erfindungsgemäß vorteilhaft in einer Konzentration von 0,001 bis 1,0 Gewichts-% bezogen auf das Gesamtgewicht der Zubereitung enthalten sein.

Die erfindungsgemäßen kosmetischen Zubereitungen können eine Reihe von Pigmenten, Farbstoffen und auch Perlglanzagentien als mattierende und/oder (leicht) überdeckende Inhaltsstoffe enthalten.

Die Farbstoffe und -pigmente können aus der entsprechenden Positivliste der Kosmetikverordnung bzw. der EG-Liste kosmetischer Färbemittel ausgewählt werden. In den meisten Fällen sind sie mit den für Lebensmittel zugelassenen Farbstoffen identisch. Vorteilhafte Farbpigmente sind beispielsweise Titandioxid, Glimmer, Eisenoxide (z. B.

$Fe_2O_3$, $Fe_3O_4$, FeO(OH)) und/oder Zinnoxid. Vorteilhafte Farbstoffe sind beispielsweise Carmin, Berliner Blau, Chromoxidgrün, Ultramarinblau und/oder Manganviolett. Es ist insbesondere vorteilhaft, die Farbstoffe und/oder Farbpigmente aus der folgenden Liste zu wählen. Die Colour Index Nummern (CIN) sind dem Rowe Colour Index, 3. Auflage, Society of Dyers and Colourists, Bradford, England, 1971 entnommen.

| **Chemische oder sonstige Bezeichnung** | **CIN** | **Farbe** |
|---|---|---|
| Pigment Green | 10006 | Grün |
| Acid Green 1 | 10020 | Grün |
| 2,4-Dinitrohydroxynaphthalin-7-sulfosäure | 10316 | Gelb |
| Pigment Yellow 1 | 11680 | Gelb |
| Pigment Yellow 3 | 11710 | Gelb |
| Pigment Orange 1 | 11725 | Orange |
| 2,4-Dihydroxyazobenzol | 11920 | Orange |
| Solvent Red 3 | 12010 | Rot |
| 1-(2'-Chlor-4'-nitro-1'-phenylazo)-2-hydroxynaphthalin | 12085 | Rot |
| Pigment Red 3 | 12120 | Rot |
| Ceresrot; Sudanrot; Fettrot G | 12150 | Rot |
| Pigment Red 112 | 12370 | Rot |
| Pigment Red 7 | 12420 | Rot |
| Pigment Brown 1 | 12480 | Braun |
| 4-(2'-Methoxy-5'-sulfosäurediethylamid-1'-phenylazo)-3-hydroxy-5''-chloro-2'',4''-dimethoxy-2-naphthoesäureanilid | 12490 | Rot |
| Disperse Yellow 16 | 12700 | Gelb |
| 1-(4-Sulfo-1-phenylazo)-4-amino-benzol-5-sulfosäure | 13015 | Gelb |
| 2,4-Dihydroxy-azobenzol-4'-sulfosäure | 14270 | Orange |
| 2-(2,4-Dimethylphenylazo-5-sulfosäure)-1-hydrooynaphthalin-4-sulfosäure | 14700 | Rot |
| 2-(4-Sulfo-1-naphthylazo)-1-naphthol-4-sulfosäure | 14720 | Rot |
| 2-(6-Sulfo-2,4-xylylazo)-1-naphthol-5-sulfosäure | 14815 | Rot |
| 1-(4'-Sulfophenylazo)-2-hydrooynaphthalin | 15510 | Orange |
| 1-(2-Sulfosäure-4-chlor-5-carbonsäure-1-phenylazo)-2-hydroxynaphthalin | 15525 | Rot |
| 1-(3-Methyl-phenylazo-4-sulfosäure)-2-hydrooynaphthalin | 15580 | Rot |
| 1-(4',(8')-Sulfosäurenaphthylazo)-2-hydrooynaphthalin | 15620 | Rot |
| 2-Hydrooy-1,2'-azonaphthalin-1'-sulfosäure | 15630 | Rot |
| 3-Hydroxy-4-phenylazo-2-naphthylcarbonsäure | 15800 | Rot |
| 1-(2-Sulfo-4-methyl-1-phenylazo)-2-naphthylcarbonsäure | 15850 | Rot |
| 1-(2-Sulfo-4-methyl-5-chlor-1-phenylazo)-2-hydrooy-naphthalin-3-carbonsäure | 15865 | Rot |
| 1-(2-Sulfo-1-naphthylazo)-2-hydrooynaphthalin-3-carbonsäure | 15880 | Rot |
| 1-(3-Sulfo-1-phenylazo)-2-naphthol-6-sulfosäure | 15980 | Orange |
| 1-(4-Sulfo-1-phenylazo)-2-naphthol-6-sulfosäure | 15985 | Gelb |
| Allura Red | 16035 | Rot |
| 1-(4-Sulfo-1-naphthylazo)-2-naphthol-3,6-disulfosäure | 16185 | Rot |
| Acid Orange 10 | 16230 | Orange |
| 1-(4-Sulfo-1-naphthylazo)-2-naphthol-6,8-disulfosäure | 16255 | Rot |
| 1-(4-Sulfo-1-naphthylazo)-2-naphthol-3,6,8-trisulfosäure | 16290 | Rot |
| 8-Amino-2 -phenylazo- 1 -naphthol-3,6-disulfosäure | 17200 | Rot |
| Acid Red 1 | 18050 | Rot |
| Acid Red 155 | 18130 | Rot |
| Acid Yellow 121 | 18690 | Gelb |
| Acid Red 180 | 18736 | Rot |
| Acid Yellow 11 | 18820 | Gelb |
| Acid Yellow 17 | 18965 | Gelb |
| 4-(4-Sulfo-1-phenylazo)-1-(4-sulfophenyl)-5-hydroxy-pyrazolon-3-carbonsäure | 19140 | Gelb |

(fortgesetzt)

| Chemische oder sonstige Bezeichnung | CIN | Farbe |
|---|---|---|
| Pigment Yellow 16 | 20040 | Gelb |
| 2,6-(4'-Sulfo-2", 4"-dimethyl)-bis-phenylazo)1,3-dihydroxy-benzol | 20170 | Orange |
| Acid Black 1 | 20470 | Schwarz |
| Pigment Yellow 13 | 21100 | Gelb |
| Pigment Yellow 83 | 21108 | Gelb |
| Solvent Yellow | 21230 | Gelb |
| Acid Red 163 | 24790 | Rot |
| Acid Red 73 | 27290 | Rot |
| 2-[4'-(4"-Sulfo-1 "-phenylazo)-7'-sulfo-1'-naphthylazo]-1-hydroxy-7-aminonaphthalin-3,6-disulfosäure | 27755 | Schwarz |
| 4'-[(4"-Sulfo-1"-phenylazo)-7'-sulfo-1'-naphthylazo]-1-hydroxy-8-acetyl-aminonaphthalin-3,5-disulfosäure | 28440 | Schwarz |
| Direct Orange 34, 39, 44, 46, 60 | 40215 | Orange |
| Food Yellow | 40800 | Orange |
| trans-β-Apo-8'-Carotinaldehyd ($C_{30}$) | 40820 | Orange |
| trans-Apo-8'-Carotinsäure ($C_{30}$)-ethylester | 40825 | Orange |
| Canthaxanthin | 40850 | Orange |
| Acid Blue 1 | 42045 | Blau |
| 2,4-Disulfo-5-hydroxy-4'-4"-bis-(diethylamino)triphenyl- | 42051 | Blau |
| carbinol 4-[(-4-N-Ethyl-p-sulfobenzylamino)-phenyl-(4-hydroxy-2-sulfo-phenyl)-(methylen)-1-(N-ethylN-p-sulfobenzyl)-2,5-cyclohexadienimin] | 42053 | Grün |
| Acid Blue 7 | 42080 | Blau |
| (N-Ethyl-p-sulfobenzyl-amino)-phenyl-(2-sulfophenyl)-methylen-(N-ethyl-N-p-sulfo-benzyl) $\Delta^{2,5}$-cyclohexadienimin | 42090 | Blau |
| Acid Green 9 | 42100 | Grün |
| Diethyl-di-sulfobenzyl-di-4-amino-2-chlor-di-2-methyl-fuchsonimmonium | 42170 | Grün |
| Basic Violet 14 | 42510 | Violett |
| Basic Violet 2 | 42520 | Violett |
| 2'-Methyl-4'-(N-ethyl-N-m-sulfobenzyl)-amino-4"-(N-diethyl)-amino-2-methyl-N-ethylN-m-sulfobenzyl-fuchsonimmonium | 42735 | Blau |
| 4'-(N-Dimethyl)-amino-4"-(N-phenyl)-aminonaphtho-N-dimethyl-fuchsonimmonium | 44045 | Blau |
| 2-Hydroxy-3,6-disulfo-4,4'-bis-dimethylamino-naphthofuchsonimmonium | 44090 | Grün |
| Acid Red 52 | 45100 | Rot |
| 3-(2'-Methylphenylamino)-6-(2'-methyl-4'-sulfophenylamino)-9-(2"-carboxyphenyl)-xantheniumsalz | 45190 | Violett |
| Acid Red 50 | 45220 | Rot |
| Phenyl-2-oxyfluoron-2-carbonsäure | 45350 | Gelb |
| 4,5-Dibromfluorescein | 45370 | Orange |
| 2,4,5,7-Tetrabromfluorescein | 45380 | Rot |
| Solvent Dye | 45396 | Orange |
| Acid Red 98 | 45405 | Rot |
| 3',4',5',6'-Tetrachlor-2,4,5,7-tetrabromfluorescein | 45410 | Rot |
| 4,5-Diiodfluorescein | 45425 | Rot |
| 2,4,5,7-Tetraiodfluorescein | 45430 | Rot |
| Chinophthalon | 47000 | Gelb |
| Chinophthalon-disulfosäure | 47005 | Gelb |

Es kann ferner günstig sein, als Farbstoff eine oder mehrer Substanzen aus der folgenden Gruppe zu wählen: 2,4-Dihydroxyazobenzol, 1-(2'-Chlor-4'-nitro-1'-phenylazo)-2-hydroxynaphthalin, Ceresrot, 2-(4-Sulfo-1-naphthylazo)-1-naphthol-4-sulfosäure, Calciumsalz der 2-Hydroxy-1,2'-azonaphthalin-1'-sulfosäure, Calcium- und Bariumsalze der 1-(2-Sulfo-4-methyl-1-phenylazo)-2-naphthylcarbonsäure, Calciumsalz der 1-(2-Sulfo-1-naphthylazo)-2-hydroxynaphthalin-3-carbonsäure, Aluminiumsalz der 1-(4-Sulfo-1-phenylazo)-2-naphthol-6-sulfosäure, Aluminiumsalz der 1-(4-Sulfo-1-naphthylazo)-2-naphthol-3,6-disulfosäure, 1-(4-Sulfo-1-naphthylazo)-2-naphthol-6,8-disulfosäure, Aluminiumsalz der 8-Amino-2 -phenylazo-1-naphthol-3,6-disulfosäure , Aluminiumsalz der 4-(4-Sulfo-1-phenylazo)-1-(4-sulfophenyl)-5-hydroxy-pyrazolon-3-carbonsäure, 4'-[(4''-Sulfo-1''-phenylazo)-7'-sulfo-1'-naphthylazo]-1-hydroxy-8-acetyl-aminonaphthalin-3,5-disulfosäure, Aluminium- und Zirkoniumsalze von 4,5-Dibromfluorescein, Aluminium- und Zirkoniumsalze von 2,4,5,7-Tetrabromfluorescein, 3',4',5',6'-Tetrachlor-2,4,5,7-tetrabromfluorescein und sein Aluminiumsalz, Aluminiumsalz von 2,4,5,7-Tetraiodfluorescein, Aluminiumsalz der Chinophthalon-disulfosäure, Aluminiumsalz der Indigodisulfosäure, 4,4'-Dimethyl-6,6'-dichlorthioindigo, Komplexsalz (Na, Al, Ca) der Karminsäure, rotes und schwarzes Eisenoxid (CIN: 77 491 (rot) und 77 499 (schwarz)), Eisenoxidhydrat (CIN: 77 492), Manganammoniumdiphosphat (CIN 77745), Ultramarin (CIN 77007) und Titandioxid.

Erfindungsgemäße Zubereitungen können Titandioxide enthalten, die sowohl in der Kristallmodifikation Rutil als auch Anatas vorliegen können und im Sinne der vorliegenden Erfindung vorteilhaft oberflächlich behandelt ("gecoatet") ist, wobei beispielsweise ein hydrophiler, amphiphiler oder hydrophober Charakter gebildet werden bzw. erhalten bleiben soll. Diese Oberflächenbehandlung kann darin bestehen, dass die Pigmente nach an sich bekannten Verfahren mit einer dünnen hydrophilen und/oder hydrophoben anorganischen und/oder organischen Schicht versehen werden. Die verschiedenen Oberflächenbeschichtungen können im Sinne der vorliegenden Erfindung auch Wasser enthalten.

Anorganische Oberflächenbeschichtungen im Sinne der vorliegenden Erfindung können bestehen aus Aluminiumoxid ($Al_2O_3$), Aluminiumhydroxid $Al(OH)_3$, bzw. Aluminiumoxidhydrat (auch: Alumina, CAS-Nr.: 1333-84-2), Natriumhexametaphosphat $(NaPO_3)_6$, Natriummetaphosphat $(NaPO_3)_n$, Siliciumdioxid ($SiO_2$) (auch: Silica, CAS-Nr.: 7631-86-9), Zirkoniumoxid ($ZrO_2$) oder Eisenoxid ($Fe_2O_3$). Diese anorganischen Oberflächenbeschichtungen können allein, in Kombination und/oder in Kombination mit organischen Beschichtungsmaterialien vorkommen.

Hierzu werden Oxide, Oxidhydrate oder Phosphate beispielsweise der Elemente Al, Si, Zr in dichten Schichten auf die Pigmentoberfläche aufgefällt.

Die anorganische Nachbehandlung geschieht im allgemeinen in einer wässrigen Suspension des Pigmentes durch Zugabe löslicher Nachbehandlungschemikalien, wie z. B. Aluminiumsulfat, und anschließende Ausfällung des im neutralen Bereich schwerlöslichen Hydroxides durch gezielte Einstellung des pH-Wertes mit Natronlauge.

Nach der anorganischen Nachbehandlung werden die gecoateten Pigmente durch Filtration aus der Suspension abgetrennt und sorgfältig gewaschen, um die gelösten Salze zu entfernen, anschließend werden die isolierten Pigmente getrocknet.

Besonders bevorzugt im Sinne dieser Erfindung sind Titandioxide, auf die Aluminiumhydroxid auf die Oberfläche aufgebracht worden ist, wie z. B. die von Sun Chemical erhältlichen Titandioxid Typen C47-051 und C47- 5175. Weiterhin bevorzugte Pigmente sind Titandioxide, die mit Aluminium- und / oder Siliziumoxiden gecoated sind, wie z. B. von der Firma Krosnos Titan: Kronos 1071 und 1075 oder von der Firma Kingfisher: A310.03 Tudor Aspen.

Organische Oberflächenbeschichtungen im Sinne der vorliegenden Erfindung können bestehen aus pflanzlichem oder tierischem Aluminiumstearat, pflanzlicher oder tierischer Stearinsäure, Laurinsäure, Dimethylpolysiloxan (auch: Dimethicone), Methylpolysiloxan (Methicone), Simethicone (einem Gemisch aus Dimethylpolysiloxan mit einer durchschnittlichen Kettenlänge von 200 bis 350 Dimethylsiloxan-Einheiten und Silicagel) oder Alginsäure. Diese organischen Oberflächenbeschichtungen können allein, in Kombination und/oder in Kombination mit anorganischen Beschichtungsmaterialien vorkommen.

Die Farbstoffe und Pigmente können sowohl einzeln als auch im Gemisch vorliegen sowie gegenseitig miteinander beschichtet sein, wobei durch unterschiedliche Beschichtungsdicken im allgemeinen verschiedene Farbeffekte hervorgerufen werden. Die Gesamtmenge der Farbstoffe und farbgebenden Pigmente wird vorteilhaft aus dem Bereich von z. B. 0,1 Gew.-% bis 30 Gew.-%, vorzugsweise von 0,5 bis 15 Gew.-%, insbesondere von 1,0 bis 10 Gew.-% gewählt, jeweils bezogen auf das Gesamtgewicht der Zubereitungen.

Die Liste der genannten Substanzen zur Mattierung bzw. zum Überdecken, die im Sinne der vorliegenden Erfindung eingesetzt werden können, soll selbstverständlich nicht limitierend sein. Ebenso ist es selbstverständlich, dass die einzelnen Substanzen zur Mattierung und/oder (leichten) Überdeckung (Farbstoffe, Pigmente usw.) einzeln wie auch in unterschiedlichen Kombinationen aus zwei oder mehreren verwendet werden können.

**Beispiele:**

Die nachfolgenden Beispiele sollen die vorliegende Erfindung verdeutlichen, ohne sie einzuschränken. Die Zahlenwerte in den Beispielen bedeuten Gewichtsprozente, bezogen auf das Gesamtgewicht der jeweiligen Zubereitungen.

**Beispielrezepturen 1-5 (Basisschutz):** (O/W-Cremes mit SPF 5-15)

| **Beispiel Nummer** | **1** |
|---|---|
| **SPF** | **5** |
| Glycerylstearatcitrat | 2 |
| Myristylmyristat | 1 |
| Cetylalkohol | 2 |
| Butylenglycol Dicaprylat/Dicaprat | 1 |
| Octamethyltetrasiloxan (Cyclomethicon) | 4 |
| Dicaprylylether | 1 |
| Ethylhexylmethoxycinnamat | 3 |
| Butyl Methoxydibenzoylmethan | 1 |
| Ethylhexylsalicylat | 3 |
| Ubichinon (Q10) | 0,05 |
| Natriumascorbylphosphat | 0,1 |
| Iminodisuccinat, Natriumsalz | 0,2 |
| Phenoxyethanol | 0,3 |
| p-Hydroxybenzoesäurealkylester (Paraben) | 0,6 |
| Diazolidinylharnstoff | 0,25 |
| Erythrulose | 2,5 |
| Xanthan Gummi | 0,1 |
| Polyacrylsäure (Carbomer) | 0,05 |
| Glycerin | 10 |
| Butylenglycol | 2 |
| Füllstoffe/ Additive (Distärke-phosphat, $SiO_2$, BHT, Talkum, Aluminiumstearat) | 0,1 |
| Parfüm | q.s. |
| Wasser | Ad 100 |

| **Beispiel Nummer** | **2** |
|---|---|
| **SPF** | **15** |
| Glycerylstearat, selbstemulgierend | 5 |
| Stearylalkohol | 1 |
| C12-15 Alkylbenzoat | 3 |
| Dicaprylylether | 4 |
| Ethylhexylmethoxycinnamat | 5 |
| Ethylhexylcyanodiphenyl-acrylat | 5 |
| Benzophenone-3 | 3 |
| Trinatrium EDTA | 0.1 |
| p-Hydroxybenzoesäurealkylester (Paraben) | 0,3 |
| Hexamidindiisethionat | 0,04 |

(fortgesetzt)

| Beispiel Nummer | 2 |
|---|---|
| **SPF** | **15** |
| 1,3-Dimethylol-5,5-dimethyl-hydantoin (DMDM Hydantoin) | 0,2 |
| Iodopropynylbutylcarbamat | 0,1 |
| Ethanol denaturiert | 2 |
| Polyacrylamid | 0,2 |
| Butylenglycol | 5 |
| Füllstoffe/ Additive (Distärke-phosphat, $SiO_2$, BHT, Talkum, Aluminiumstearat) | 1 |
| Parfüm | q.s. |
| Wasser | Ad 100 |

| Beispiel Nummer | 3 | 4 |
|---|---|---|
| **SPF** | **10** | **8** |
| Glycerylstearatcitrat | | 2 |
| Glycerylstearat (selbstemulgierend) | 3 | |
| PEG-40-Stearat | 1 | |
| Cetearylalkohol | | 4 |
| Cetylalkohol | 3 | |
| C12-15 Alkylbenzoat | 2 | |
| Butylenglycol Dicaprylat/Dicaprat | | 1 |
| Octyldodecanol | 1 | |
| Dimethylpolysiloxan (Dimethicon) | 1 | 1 |
| Dicaprylylether | 2 | |
| Dicarprylylcarbonat | | 3 |
| $TiO_2$ | 2 | 3 |
| Ethylhexylmethoxycinnamat | 5 | |
| Bis-Imidazylate | 2 | |
| Phenylbenzimidazole Sulfonic Acid | 2 | |
| Bis-Ethylhexyloxyphenol-methoxyphenyltriazine | | 2 |
| Ethylhexylsalicylat | | 5 |
| Tocopherylacetat | 1 | |
| Trinatrium EDTA | | 0,2 |
| Phenoxyethanol | 0,3 | 0,2 |
| p-Hydroxybenzoesäurealkylester (Paraben) | 0,2 | 0,3 |
| Dihydroxyaceton | 1,5 | |
| Polyacrylsäure (Carbomer) | 0,1 | |
| Glycerin | 6 | 7,5 |
| Füllstoffe/ Additive (Distärke-phosphat, $SiO_2$, BHT, Talkum, Aluminiumstearat) | 0,2 | 0,5 |

(fortgesetzt)

| **Beispiel Nummer** | **3** | **4** |
|---|---|---|
| **SPF** | **10** | **8** |
| Parfüm | q.s. | q.s. |
| Wasser | Ad 100 | Ad 100 |

| **Beispiel Nummer** | **5** |
|---|---|
| **SPF** | **12** |
| Glycerylstearat (selbstemulgierend) | 2 |
| PEG-40-Stearat | 1 |
| Myristylmyristat | 1 |
| Cetearylalkohol | 2 |
| C12-15 Alkylbenzoat | 3 |
| Ethylhexylmethoxycinnamat | 5 |
| Benzophenone-3 | 3 |
| Ethylhexylsalicylat | 4 |
| Iminodisuccinat, Natriumsalz | 0,1 |
| Phenoxyethanol | 0,2 |
| p-Hydroxybenzoesäurealkylester (Paraben) | 0,3 |
| Polyacrylsäure (Carbomer) | 0,1 |
| Glycerin | 8 |
| Füllstoffe/ Additive (Distärke-phosphat, $SiO_2$, BHT, Talkum, Aluminiumstearat) | 0,05 |
| Parfüm | q.s. |
| Wasser | Ad 100 |

**Beispiel 6 (Basisschutz)**

O/W Selbstbräunungsemulsion mit Dihydroxyacetone und SPF 10

| | |
|---|---|
| Aqua | Ad 100 |
| Polyglyceryl-3 Methylglucose Distearate | 5 |
| Butylene Glycol Dicaprylate/Dicaprate | 4 |
| Octyldodecanol | 3 |
| Citric Acid | 0,2 |
| Glycerin | 5 |
| Butylene Glycol | 3 |
| Phenoxyethanol | 0,3 |
| Methylparaben | 0,4 |
| Propylparaben | 0,1 |

(fortgesetzt)

| | |
|---|---|
| Sodium Hydroxide | q.s. |
| Dihydroxyacetone | 2,5 |
| Ethylhexyl Methoxycinnamate | 4 |
| Benzophenone- 3 | 3 |
| Octocrylene | 2 |
| Xanthan Gum | 0,2 |

**Beispiel 7 (Fleckschutz)**

Wasserfreier Wachsstift zur kleinflächigen Applikation (SPF 20)

| | |
|---|---|
| Octyldodecanol | 25 |
| Cetyl Alcohol | 3 |
| Cetyl Palmitate | 18,8 |
| Myristyl Myristate | 5 |
| Butyl Methoxydibenzoylmethane | 3 |
| Ethylhexylmethoxycinnamate | 7,5 |
| Octocrylene | 5 |
| Cera Alba | 3 |
| Chamomilla Recutita | 0,2 |
| Hydrogenated Polydecene | 8 |
| Cera Microcristallina | 22 |

**Beispiel 8 (Fleckschutz)**

W/O Emulsionen zur kleinflächigen Applikation (im Stift mit Schwämmchenapplikator) SPF 30

| | |
|---|---|
| Aqua | 58 |
| Stearic Acid | 0,5 |
| Dimethicone | 3,5 |
| Cyclomethicone | 1 |
| Cera Microcristallina | 0,5 |
| Paraffinum Liquidum | 1 |
| Syntetic Beeswax | 2 |
| Octyldodecanol | 3 |
| Glycerin | 2 |
| Propylene Glycol | 1 |
| Titanium Dioxide | 4 |
| Ethylhexylmethoxycinnamate | 7,5 |

(fortgesetzt)

| | |
|---|---|
| Octylsalicylate | 5 |
| Bis-Ethylhexyloxyphenolmethoxyphenyltriazine | 3 |
| Phenylbenzimidazole Sulfonic Acid | 2 |
| PEG-40 Stearate | 2 |
| Cetyl Alcohol | 1 |
| Myreth 4 | 0,5 |
| Ceteareth 25 | 0,5 |
| Tocopheryl Acetate | 0,5 |
| Tetrasodium Imidosuccinate | 0,1 |
| Xanthan Gum | 0,3 |
| Phenoxyethanol | 0,6 |
| Isoporoylparabene | 0,2 |
| Butylparabene | 0,1 |
| Methylparabene | 0,3 |

**Beispiel 9 (Fleckschutz)**

O/W Emulsion zur kleinflächigen Applikation (Stift mit Pinselapplikator) SPF 40

| | |
|---|---|
| Aqua | Ad 100 |
| Caprylic/Capric Triglyceride | 8 |
| Octyldodecanol | 2 |
| Glycerin | 4 |
| Stearyl Alcohol | 2 |
| Glyceryl Stearate SE | 1 |
| Trisodium EDTA | 1 |
| Ethylhexylmethoxycinnamate | 8 |
| Phenylbenzimidazo Sulfonsäure | 2 |
| Ethylhexyl Triazone | 3,5 |
| Titanium Dioxide | 6 |
| Diethylhexyl Butamido Triazone | 3 |
| Bis-Ethylhexyloxyphenyol Methoxyphenyl Triazine | 2 |
| Xanthan Gum | 0,5 |
| Sodium Hydroxide | q.s. |
| Hydrogenated Coco-Glycerides | 2 |
| Pheoxyethanol | 0,4 |
| Cyclomethicone | 4 |
| Cetyl Alcohol | 1 |
| Peg-40 Castor Oil | 1 |

(fortgesetzt)

| | |
|---|---|
| Sodium Cetearyl Sulfate | 0,5 |
| DMDM Hydantoin | 0,3 |

**Beispiel 10 (Fleckschutz)**

O/W Emulsion zur kleinflächigen Applikation (Stift mit Pinselapplikator oder mit schwämmchenapplikator), SPF 50+

| Beispiel | 10a |
|---|---|
| Alcohol Denat. | 4 |
| Glyceryl Stearate SE | 1 |
| Cetearyl Alcohol + PEG-40 Castor Oil + Sodium Cetearyl Sulfate | 2,5 |
| VP/Hexadecene Copolymer | 0,5 |
| Trisodium EDTA | 1 |
| Preservatives | q.s. |
| Aminobenzophenon | 1 |
| Phenylbenzimidazole Sulfonic Acid | 2 |
| Diethylhexyl Butamido Triazone | 2 |
| Bis-Ethylhexyloxyphenol Methoxyphenyl Triazine | 3 |
| Ethylhexyl Triazone | 4 |
| Butyl Methoxydibenzoylmethane | 4,0 |
| Ethylhexyl Methoxycinnamate | 7,5 |
| Glycerin | 7,5 |
| Hydrogenated Coco-Glycerides | 1 |
| Dicaprylyl Carbonate | 1 |
| Butylene Glycol Dicaprylate/Dicaprate | 6,5 |
| C12-15 Alkyl Benzoate | 0 |
| Parfum | q.s. |
| Titanium Dioxide | 5 |
| Xanthan Gum | 0,2 |
| C18-36 Acid Triglyceride | 1 |
| Cetyl Alcohol | 1 |
| Aqua | ad 100 |
| Tocopheryl Acetate | 0,5 |
| 8-Hexadecen-1,16-dicarbonsäure | 1 |

| Beispiel | 10b | 10c |
|---|---|---|
| Alcohol Denat. | | 2 |
| Glyceryl Stearate SE | 1,5 | 2 |

(fortgesetzt)

| Beispiel | 10b | 10c |
|---|---|---|
| Cetearyl Alcohol + PEG-40 Castor Oil + Sodium Cetearyl Sulfate | 3,75 | 5 |
| VP/Hexadecene Copolymer | 0,5 | 0,8 |
| Trisodium EDTA | 1 | 0 |
| Preservatives | q.s. | q.s. |
| 2,4-bis-[5-1(dimethylpropyl)benzoxazol-2-yl-(4-phenyl)imino]-6-(2- ethylhexyl)-imino-1,3,5-triazin | 1,5 | |
| Phenylbenzimidazole Sulfonic Acid | 2 | 2 |
| Disodium Phenyl Dibenzimidazole Tetrasulfonate | 1,5 | |
| Diethylhexyl Butamido Triazone | 1 | 3 |
| Bis-Ethylhexyloxyphenol Methoxyphenyl Triazine | 4 | 3 |
| Ethylhexyl Triazone | 4 | 1,5 |
| Butyl Methoxydibenzoylmethane | 3,0 | 4,5 |
| Ethylhexyl Methoxycinnamate | 7,5 | 0 |
| Glycerin | 4 | 7,5 |
| Hydrogenated Coco-Glycerides | 0,5 | 1 |
| Dicaprylyl Carbonate | 2 | 3 |
| Butylene Glycol Dicaprylate/Dicaprate | 7,5 | 9 |
| C12-15 Alkyl Benzoate | 0 | 5 |
| Parfum | q.s. | q.s. |
| Titanium Dioxide | 5 | 5 |
| Xanthan Gum | 0,4 | 0 |
| C18-36 Acid Triglyceride | 0,5 | 0 |
| Cetyl Alcohol | 1,5 | 1,2 |
| Aqua | ad 100 | ad 100 |
| Tocopheryl Acetate | 0,5 | 0,5 |
| Creatine | 0,5 | |
| Creatinine | 0,05 | |

| Beispiel | 10d | 10e |
|---|---|---|
| Alcohol Denat. | 4 | 4 |
| Glyceryl Stearate SE | 2 | 1,2 |
| Cetearyl Alcohol + PEG-40 Castor Oil + Sodium Cetearyl Sulfate | 5 | 2,9 |
| VP/Hexadecene Copolymer | 0,7 | 0 |
| Trisodium EDTA | 1 | 1,5 |
| Preservatives | q.s. | q.s. |
| Phenylbenzimidazole Sulfonic Acid | 2 | 2 |
| Diethylhexyl Butamido Triazone | 3 | 2 |

(fortgesetzt)

| Beispiel | 10d | 10e |
|---|---|---|
| Bis-Ethylhexyloxyphenol Methoxyphenyl Triazine | 3 | 3 |
| Ethylhexyl Triazone | 0,5 | 1,5 |
| Butyl Methoxydibenzoylmethane | 4,5 | 4,5 |
| Ethylhexyl Methoxycinnamate | 0 | 0 |
| Glycerin | 6 | 5 |
| Hydrogenated Coco-Glycerides | 0,5 | 0,6 |
| Dicaprylyl Carbonate | 4 | 2 |
| Butylene Glycol Dicaprylate/Dicaprate | 6 | 8 |
| C12-15 Alkyl Benzoate | 4 | 2 |
| Parfum | q.s. | q.s. |
| Titanium Dioxide | 5 | 5 |
| Xanthan Gum | 0,3 | 0,1 |
| C18-36 Acid Triglyceride | 1 | 0,2 |
| Cetyl Alcohol | 0,5 | |
| Aqua | ad 100 | ad 100 |
| Tocopheryl Acetate | 0,5 | 0,5 |
| Creatine | 0,5 | |
| Creatinine | 0,01 | |
| 8-Hexadecen-1,16-dicarbonsäure | 0,1 | |

| Beispiel | 10f | 10g |
|---|---|---|
| Alcohol Denat. | 0 | 3 |
| Glyceryl Stearate SE | 2 | 1,2 |
| Cetearyl Alcohol + PEG-40 Castor Oil + Sodium Cetearyl Sulfate | 5 | 2,9 |
| VP/Hexadecene Copolymer | 0,7 | 0,2 |
| Trisodium EDTA | 1 | 1 |
| Preservatives | q.s. | q.s. |
| Octocrylene | 10 | 10 |
| Terephthalidene Dicamphor Sulfonic Acid | 8 | 9 |
| Drometrizole Trisiloxane | 8 | 14 |
| Diethylhexyl Butamido Triazone | | 5 |
| Bis-Ethylhexyloxyphenol Methoxyphenyl Triazine | | 3 |
| Ethylhexyl Triazone | | 2 |
| Butyl Methoxydibenzoylmethane | 4,9 | 4,5 |
| Glycerin | 6 | 0 |
| Hydrogenated Coco-Glycerides | 0,5 | 0,6 |
| Dicaprylyl Carbonate | 4 | 2 |

(fortgesetzt)

| Beispiel | 10f | 10g |
|---|---|---|
| Butylene Glycol Dicaprylate/Dicaprate | 6 | 8 |
| C12-15 Alkyl Benzoate | 4 | 2 |
| Parfum | q.s. | q.s. |
| Titanium Dioxide | 10 | 10 |
| Xanthan Gum | 0,3 | 0,1 |
| C18-36 Acid Triglyceride | 1 | 0,2 |
| Cetyl Alcohol | 0,5 | |
| Aqua | ad 100 | ad 100 |
| Tocopheryl Acetate | 0,5 | 0,5 |
| 8-Hexadecen-1,16-dicarbonsäure | 0,1 | |

| Beispiel | 10h | 10i |
|---|---|---|
| Alcohol Denat. | 4 | 4 |
| Glyceryl Stearate SE | 1,2 | 1,2 |
| Cetearyl Alcohol + PEG-40 Castor Oil + Sodium Cetearyl Sulfate | 2,9 | 2,9 |
| VP/Hexadecene Copolymer | 0,2 | 0,2 |
| Trisodium EDTA | 1 | 1 |
| Preservatives | q.s. | q.s. |
| 2,4-bis-[5-1 (dimethylpropyl)benzoxazol-2-yl-(4-phenyl)-imino]-6-(2-ethylhexyl)-imino-1,3,5-triazin | 9,4 | 8,2 |
| Aminobenzophenon | 6,7 | 8,9 |
| Octocrylene | 10 | 10 |
| Terephthalidene Dicamphor Sulfonic Acid | 9 | 9 |
| Drometrizole Trisiloxane | 14 | 14 |
| Diethylhexyl Butamido Triazone | 5 | 5 |
| Bis-Ethylhexyloxyphenol Methoxyphenyl Triazine | 3 | 3 |
| Ethylhexyl Triazone | 2 | 2 |
| Butyl Methoxydibenzoylmethane | | 1 |
| Glycerin | 5 | 5 |
| Hydrogenated Coco-Glycerides | 0,6 | 0,6 |
| Dicaprylyl Carbonate | 2 | 2 |
| Butylene Glycol Dicaprylate/Dicaprate | 8 | 8 |
| C12-15 Alkyl Benzoate | 2 | 2 |
| Parfum | q.s. | q.s. |
| Titanium Dioxide | 10 | 10 |
| Xanthan Gum | 0,1 | 0,1 |
| C18-36 Acid Triglyceride | 0,2 | 0,2 |

(fortgesetzt)

| Beispiel | 10h | 10i |
|---|---|---|
| Aqua | ad 100 | ad 100 |
| Tocopheryl Acetate | 0,5 | 0,5 |

## Patentansprüche

1. Kosmetisches Kit zur Reduktion von Hautpigmentierungsunterschieden, **dadurch gekennzeichnet, dass** ein Sonnenschutzmittel zum Basisschutz und ein Sonnenschutzmittel zum Fleckschutz aufweist, wobei der Lichtschutzfaktor des Sonnenschutzmittels zum Basisschutzes niedriger ist als der Lichtschutzfaktor des Sonnenschutzmittels zum Fleckschutz.

2. Kosmetisches Kit nach Anspruch 1, **dadurch gekennzeichnet, dass** der Unterschied der Lichtschutzfaktoren der beiden Sonnenschutzmittel um mindestens 10 LSF Einheiten, bevorzugt mindestens 15 LSF Einheiten, besonders bevorzugt um mindestens 20 LSF-Einheiten beträgt.

3. Kosmetisches Kit nach mindestens einem der Ansprüche 1 bis 2, **dadurch gekennzeichnet, dass** das Sonnschutzmittels für den Basisschutzes einen Lichtschutzfaktor von mindestens 5, bevorzugt mindestens 8, besonders bevorzugt mindestens 10 aufweisen, jedoch nicht mehr als LSF30 aufweist.

4. Kosmetisches Kit nach mindestens einem der Ansprüche 1 bis 3, **dadurch gekennzeichnet, dass** das Verhältnis der Lichtschutzfaktoren von Fleckschutz und Basisschutz ($LFS_{Fleckschutz}$ /$LSF_{Basisschutz}$) bei mindestens 1.5, bevorzugt mindestens 2, besonders bevorzugt mindestens 2.5 beträgt.

5. Kosmetisches Kit nach mindestens einem der Ansprüche 1 bis 4, **dadurch gekennzeichnet, dass** die UVA-Balance des Fleckschutzes mindestens 20, bevorzugt mindestens 30, besonders bevorzugt bei mindestens 40 beträgt.

6. Kosmetisches Kit nach mindestens einem der Ansprüche 1 bis 5, **dadurch gekennzeichnet, dass** die UVA-Balance des Basisschutzes mindestens 15, bevorzugt mindestens 25, besonders bevorzugt bei mindestens 35 beträgt.

7. Kosmetisches Kit nach mindestens einem der Ansprüche 1 bis 6, **dadurch gekennzeichnet, dass** der Basisschutz, ein oder mehrere selbstbräunende Substanzen enthält, insbesondere Dihydroxyaceton und/oder Erythrulose.

8. Kosmetisches Kit nach mindestens einem der Ansprüche 1 bis 7, **dadurch gekennzeichnet, dass** der Fleckschutz ein oder mehrere pigmentierungsmindernde Wirkstoffe enthält, bevorzugt 8-Hexadecen-1,16-dicarbonsäure, Liponamid, Deoxyarbutin, Arbutin, Kojisäure und/oder, Extrakte des Süssholzes.

9. Kosmetisches Kit nach mindestens einem der Ansprüche 1 bis 8, **dadurch gekennzeichnet, dass** der Basisschutz und/oder der Fleckschutz weitere kosmetischen und/oder dermatologischen Wirk-, Hilfs- und Zusatzstoffe enthält.

10. Kosmetisches Kit nach mindestens einem der Ansprüche 1 bis 9, **dadurch gekennzeichnet**, das der Basisschutz und/oder Fleckschutz Moisturizer und/oder Antioxidantien enthält.

11. Kosmetisches Kit nach mindestens einem der Ansprüche 1 bis 10, **dadurch gekennzeichnet, dass** es sich bei dem Fleckschutz und/oder Basisschutz O/W-Emulsionen, Hydrodispersionen, Pickering-Emulsionen, Stifte oder Hydrogele handelt.

12. Kosmetisches Kit nach mindestens einem der Ansprüche 1 bis 10, **dadurch gekennzeichnet, dass** der Basisschutz und/oder Fleckschutz einen oder mehrere Puderrohstoffe und/oder ein oder mehrere Zuckerderivate in einer Menge von 0,5 bis 10 Gew.-%, bevorzugt in einer Menge von 2 bis 8 Gew.-% und ganz besonders bevorzugt in einer Menge von 3 bis 6 Gew.-% bezogen auf das Gesamtgewicht der Zubereitung enthält.

13. Kosmetisches Kit nach Ansprüch 12, **dadurch gekennzeichnet, dass** mindestens ein Zuckerderivat eine Stärke und/oder ein Stärkederivat und mindestens ein Zuckerderivat ein Cyclodextrin und/oder Cyclodextrinderivaten ist und das Gewichtsverhältnis der Gesamtmenge an Stärke und/oder Stärkederivaten zur Gesamtmenge an Cyclodextrinen und/oder Cyclodextrinderivaten von 1:5 bis zu 20:1 beträgt.

14. Kosmetisches Kit nach mindestens einem der Ansprüche 1 bis 10, **dadurch gekennzeichnet, dass** die kosmetische Zubereitung einen oder mehrere Farbstoffe und/oder Pigmente in einer Menge von 0,1 bis 30 Gew.-%, vorzugsweise von 0,5 bis 15 Gew.-%, insbesondere von 1,0 bis 10 Gew.-% gewählt bezogen auf das Gesamtgewicht der Zubereitung enthält.

15. Verfahren zur Reduktion des Kontrastunterschiedes nicht vollflächig pigmentierter Haut, **dadurch gekennzeichnet, dass** zuerst das anzugleichende Hautareal vollflächig mit einem ersten Sonnenschutzmittel (Basisschutz) behandelt wird und anschließend die stark pigmentierten Bereiche des anzugleichenden Hautareals selektiv mit einem zweiten Sonnenschutzmittel (Fleckschutz) behandelt werden, und anschließender Bestrahlung mit natürlicher oder künstlicher UV-Strahlung, wobei der Lichtschutzfaktor des ersten Sonnenschutzmittels kleiner ist als der des zweiten Sonnenschutzmittels.

16. Verfahren nach Anspruch 15, **dadurch gekennzeichnet, dass** der Unterschied der Lichtschutzfaktoren von Basisschutz und Fleckschutz mindestens 10 LSF-Einheiten, bevorzugt mindestens 15 LSF-Einheiten, besonders bevorzugt um mindestens 20 LSF-Einheiten beträgt.

17. Verfahren nach mindestens einem der Ansprüche 15 bis 16, **dadurch gekennzeichnet**, das das Sonnschutzmittels für den Basisschutzes einen Lichtschutzfaktor von mindestens LSF 5, bevorzugt mindestens LSF 8, besonders bevorzugt mindestens LSF 10 aufweisen, jedoch nicht mehr als LSF30 aufweist

18. Verfahren nach mindestens einem der Ansprüche 15 bis 17, **dadurch gekennzeichnet**, das das Verhältnis der Lichtschutzfaktoren von Fleckschutz und Basisschutz ($LFS_{Fleckschutz}$ /$LSF_{Basisschutz}$) bei mindestens 1.5, bevorzugt mindestens 2, besonders bevorzugt mindestens 2.5 beträgt.

19. Verfahren nach mindestens einem der Ansprüche 15 bis 18, **dadurch gekennzeichnet**, das die UVA-Balance des Fleckschutzes mindestens 20, bevorzugt mindestens 30, besonders bevorzugt bei mindestens 40 beträgt.

20. Verfahren nach mindestens einem der Ansprüche 15 bis 19, **dadurch gekennzeichnet**, das die UVA-Balance des Basisschutzes mindestens 15, bevorzugt mindestens 25, besonders bevorzugt bei mindestens 35 beträgt.

21. Verfahren nach mindestens einem der Ansprüche 15 bis 20, **dadurch gekennzeichnet, dass** es bei einer Auftragung auf die zu färbenden Hautareale nicht zum direkten Kontakt mit Händen bzw. Fingern kommt, insbesondere durch Verwendung von Stift-, Roll-on-, Spray-, Pinsel-, Bürsten-, Schwammapplikatoren.

22. Verwendung von Sonnenschutzmitteln mit unterschiedlichem Lichtschutzfaktor zur Farbangleichung von unterschiedlich pigmentierten Hautstellen von mehrzelligen Organismen, insbesondere der Haut von Mensch und Tier, wobei ein Sonnenschutzmittel vollflächig auf ein Hautareal appliziert wird (Basisschutz) und ein zweites Sonnenschutzmittel (Fleckschutz) mit einem Lichtschutzfaktor der höher ist als der Lichtschutzfaktor des Basisschutzes selektiv auf stark pigmentierte Bereiche aufgetragen wird.

23. Verwendung nach Anspruch 22, **dadurch gekennzeichnet, dass** das Sonnenschutzmittel für den Fleckschutz einen Lichtschutzfaktor von mindestens 10 LSF-Einheiten, bevorzugt mindestens 15 LSF-Einheiten, besonders bevorzugt mindestens 20 LSF-Einheiten, aufweist und das zweite Sonnenschutzmittel für den Basisschutz einen Lichtschutzfaktor von 1 bis 30 LSF-Einheiten, besonders bevorzugt 5 bis 15 LSF-Einheiten aufweist.

24. Verwendung nach mindestens einem der Ansprüche 22 bis 23, zur Kaschierung von natürlichen, unnatürlichen und/oder krankhaften Hautpigmentierungsstörungen, insbesondere von Sommersprossen (Lentigo aestiva), Altersflekken (Lentigo senilis, Keratosis senilis), Leberflecken (Lentigo simplex, Lentigo juvenilis), Lentigo maligna (Dubreuilh-Hutchinson Krankheit), Narben von beispielsweise Operationen, Verletzungen oder Verbrennungen (postinflammatorische Hyperpigmentierungen), Melasma, unregelmäßige Pigmentierung der Haut mit lokal hyper- und/oder hypopigmentierten Bereichen *(uneven skin tone)* infolge äußerer Einflüsse (insbesondere nach chronischer UV-Belastung).

25. Verwendung nach mindestens einem der Ansprüche 22 bis 24, zur Unterdrückung und/oder Verringerung des subjektiven Gefühls des ’Altwerdens’ bzw. ’Altseins’, durch Kaschierung von Altersflecken und anderen durch Alterung entstehenden Haupigmentierungsunregelmäßigkeiten.

Europäisches Patentamt

# EUROPÄISCHER TEILRECHERCHENBERICHT

der nach Regel 45 des Europäischen Patentübereinkommens für das weitere Verfahren als europäischer Recherchenbericht gilt

Nummer der Anmeldung
EP 06 11 2463

## EINSCHLÄGIGE DOKUMENTE

| Kategorie | Kennzeichnung des Dokuments mit Angabe, soweit erforderlich der maßgeblichen Teile | Betrifft Anspruch | KLASSIFIKATION DER ANMELDUNG (IPC) |
|---|---|---|---|
| X | WO 2005/004826 A (BEIERSDORF AG; WOLBER, RAINER; BATZER, JAN; BLATT, THOMAS; WENDEL, VOL) 20. Januar 2005 (2005-01-20)<br>* das ganze Dokument *<br>----- | 1-14 | INV.<br>A61K8/35<br>A61Q17/04 |
| X | US 2004/228810 A1 (HAMSON STARR ET AL) 18. November 2004 (2004-11-18)<br>* Absätze [0022] - [0029] *<br>* Anspruch 1 *<br>----- | 1-7,9-11 | |
| X | DE 198 47 936 A1 (COTY B.V) 20. April 2000 (2000-04-20)<br>* das ganze Dokument *<br>----- | 1-7,9-11 | |
| X | DE 196 03 018 A1 (LANCASTER GROUP GMBH, 67059 LUDWIGSHAFEN, DE) 14. August 1997 (1997-08-14)<br>* Spalte 1, Zeile 36 - Zeile 46 *<br>* Beispiele 1,2 *<br>* Ansprüche 1-3 *<br>-----<br>-/-- | 1-7,9-11 | |

RECHERCHIERTE SACHGEBIETE (IPC)

A61K

## UNVOLLSTÄNDIGE RECHERCHE

Die Recherchenabteilung ist der Auffassung, daß ein oder mehrere Ansprüche, den Vorschriften des EPÜ in einem solchen Umfang nicht entspricht bzw. entsprechen, daß sinnvolle Ermittlungen über den Stand der Technik für diese Ansprüche nicht, bzw. nur teilweise, möglich sind.

Vollständig recherchierte Patentansprüche:

Unvollständig recherchierte Patentansprüche:

Nicht recherchierte Patentansprüche:

Grund für die Beschränkung der Recherche:

Siehe Ergänzungsblatt C

3

| Recherchenort | Abschlußdatum der Recherche | Prüfer |
|---|---|---|
| München | 28. August 2006 | Paloniemi Legland, R |

KATEGORIE DER GENANNTEN DOKUMENTEN

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument

& : Mitglied der gleichen Patentfamilie, übereinstimmendes Dokument

EPO FORM 1503 03.82 (P04C09)

Europäisches Patentamt

## EUROPÄISCHER TEILRECHERCHENBERICHT

Nummer der Anmeldung
EP 06 11 2463

| EINSCHLÄGIGE DOKUMENTE | | | KLASSIFIKATION DER ANMELDUNG (IPC) |
|---|---|---|---|
| Kategorie | Kennzeichnung des Dokuments mit Angabe, soweit erforderlich der maßgeblichen Teile | Betrifft Anspruch | |
| X | US 5 514 437 A (TANNER ET AL)<br>7. Mai 1996 (1996-05-07)<br>* Spalte 7, Zeile 29 - Zeile 33 *<br>* Beispiele IV-VIII *<br>* Ansprüche 1,14 *<br>----- | 1-12,14 | |
| | | | RECHERCHIERTE SACHGEBIETE (IPC) |

3

Europäisches Patentamt

**UNVOLLSTÄNDIGE RECHERCHE ERGÄNZUNGSBLATT C**

Nummer der Anmeldung
EP 06 11 2463

Obwohl die Ansprüche 15-25 sich auf ein Verfahren zur Behandlung des menschlichen/tierischen Körpers beziehen (Artikel 52(4) EPÜ), wurde die Recherche durchgeführt und gründete sich auf die angeführten Wirkungen der Verbindung/Zusammensetzung.

-----

Grund für die Beschränkung der Recherche (nicht patentfähige Erfindung(en)):

Artikel 52 (4) EPÜ - Verfahren zur therapeutischen Behandlung des menschlichen oder tierischen Körpers

**ANHANG ZUM EUROPÄISCHEN RECHERCHENBERICHT ÜBER DIE EUROPÄISCHE PATENTANMELDUNG NR.** EP 06 11 2463

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

28-08-2006

| Im Recherchenbericht angeführtes Patentdokument | Datum der Veröffentlichung | Mitglied(er) der Patentfamilie | Datum der Veröffentlichung |
|---|---|---|---|
| WO 2005004826 A | 20-01-2005 | EP 1648395 A1 | 26-04-2006 |
| US 2004228810 A1 | 18-11-2004 | KEINE | |
| DE 19847936 A1 | 20-04-2000 | AT 233541 T | 15-03-2003 |
| | | WO 0021500 A1 | 20-04-2000 |
| | | EP 1119343 A1 | 01-08-2001 |
| | | ES 2189525 T3 | 01-07-2003 |
| | | HU 0103639 A2 | 28-02-2002 |
| | | JP 2002527372 T | 27-08-2002 |
| | | PL 346970 A1 | 11-03-2002 |
| | | US 6403062 B1 | 11-06-2002 |
| DE 19603018 A1 | 14-08-1997 | AT 198038 T | 15-12-2000 |
| | | AU 712748 B2 | 18-11-1999 |
| | | AU 2149597 A | 11-08-1997 |
| | | BG 102629 A | 30-07-1999 |
| | | BR 9707001 A | 20-07-1997 |
| | | CA 2240461 A1 | 24-07-1997 |
| | | CZ 9802207 A3 | 14-10-1998 |
| | | WO 9725970 A1 | 24-07-1997 |
| | | EA 809 B1 | 24-04-2000 |
| | | EP 0954277 A1 | 10-11-1999 |
| | | ES 2154896 T3 | 16-04-2001 |
| | | HU 9901358 A2 | 30-08-1999 |
| | | JP 2000503305 T | 21-03-2000 |
| | | NO 982966 A | 25-06-1998 |
| | | NZ 330884 A | 30-08-1999 |
| | | PL 327470 A1 | 07-12-1998 |
| | | SK 74198 A3 | 04-11-1998 |
| | | TR 9801169 T2 | 21-10-1998 |
| | | US 6007796 A | 28-12-1999 |
| US 5514437 A | 07-05-1996 | AT 173155 T | 15-11-1998 |
| | | CA 2186500 A1 | 05-10-1995 |
| | | DE 69505962 D1 | 17-12-1998 |
| | | DE 69505962 T2 | 24-06-1999 |
| | | DK 752842 T3 | 26-07-1999 |
| | | EP 0752842 A1 | 15-01-1997 |
| | | ES 2126887 T3 | 01-04-1999 |
| | | GR 3029353 T3 | 28-05-1999 |
| | | JP 9510969 T | 04-11-1997 |
| | | WO 9526178 A1 | 05-10-1995 |

EPO FORM P0461

Für nähere Einzelheiten zu diesem Anhang : siehe Amtsblatt des Europäischen Patentamts, Nr.12/82

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- EP 570838 A1
- EP 775698 A
- WO 9220690 A


### In der Beschreibung aufgeführte Nicht-Patentliteratur


- **V. WENDEL et al.** The influence of pre-irradiation on the predictability of in vivo UVA protection with a new in vitro method. *Photodermatol Photoimmunol Photomed,* 2003, vol. 19, 93-97
- *CHEMICAL ABSTRACTS,* 178463-23-5
- Rowe Colour Index. Society of Dyers and Colourists, 1971